(19) Europäisches Patentamt
European Patent Office
Office européen des brevets

(11) Veröffentlichungsnummer: **0 361 300**
**A1**

# (12) EUROPÄISCHE PATENTANMELDUNG

(21) Anmeldenummer: **89117391.6**

(22) Anmeldetag: **20.09.89**

(51) Int. Cl.5: **C07C 211/60 , C07C 271/30 , C07C 271/24 , C07C 233/34 , C07C 233/43 , C07D 333/20 , C07D 307/52 , C07D 213/36 , C07D 295/16 , C07C 313/20**

(30) Priorität: **24.09.88 DE 3832571**

(43) Veröffentlichungstag der Anmeldung:
**04.04.90 Patentblatt 90/14**

(84) Benannte Vertragsstaaten:
**AT BE CH DE ES FR GB GR IT LI LU NL SE**

(71) Anmelder: **BOEHRINGER INGELHEIM KG**
**Postfach 200**
**D-6507 Ingelheim am Rhein(DE)**
(84) **BE CH DE ES FR GR IT LI LU NL SE AT**

Anmelder: **BOEHRINGER INGELHEIM INTERNATIONAL GmbH**
**Postfach 20**
**D-6507 Ingelheim am Rhein(DE)**
(84) **GB**

(72) Erfinder: **Grauert, Matthias, Dr. Dipl.-Chem.**
**Eltviller Strasse 2**
**D-6507 Ingelheim am Rhein(DE)**
Erfinder: **Merz, Herbert, Dr. Dipl.-Chem.**
**Rotweinstrasse 53**
**D-6507 Ingelheim am Rhein(DE)**
Erfinder: **Mierau, Joachim, Dr.**
**An den Weiden 3**
**D-6500 Mainz 30(DE)**
Erfinder: **Schingnitz, Günter, Dr.**
**unter den Gärten 10**
**D-6550 Bad Kreunach(DE)**
Erfinder: **Schneider, Claus, Dr. Dipl.-Chem.**
**Im Konrad-Adenauer-Ring 21**
**D-6904 Eppelheim(DE)**

## (54) Verwendung von 2,7-Diamino-1,2,3,4-tetrahydronaphthalinen als Arzneimittel, neue 2,7-Diamino-1,2,3,4-tetrahydronaphtaline sowie Verfahren zu ihrer Herstellung.

(57) Die vorliegende Erfindung betrifft die Verwendung von 2,7-Diamino-1,2,3,4-tetrahydronaphthalinen als Arzneimittel, neue 2,7-Diamino-1,2,3,4-tetrahydronaphthaline sowie Verfahren zu ihrer Herstellung.

EP 0 361 300 A1

# Verwendung von 2,7-Diamino-1,2,3,4-tetrahydronaphthalinen als Arzneimittel, neue 2,7-Diamino-1,2,3,4-tetrahydronaphthaline sowie Verfahren zu ihrer Herstellung

Die Erfindung betrifft neue 2,7-Diamino-1,2,3,4-tetrahydronaphthaline, ihre Herstellung und Verwendung als Arzneimittel. Die 2,7-Diamino-1,2,3,4-tetrahydronaphthaline (2,7-Diaminotetraline) entsprechen der allgemeinen Formel I

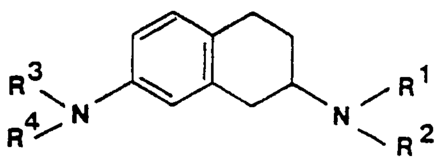

(I)

worin

$R^1$ Wasserstoff, $C_1$-$C_{12}$-Alkyl, $(CH_2)_a$-$R^5$, $CH_2$-$CH=CH$-$R^6$, Alkoxycarbonyl und a eine Zahl 1, 2, 3 oder 4;

$R^2$ Wasserstoff, $C_1$-$C_{12}$-Alkyl, $(CH_2)_b$-$R^7$, Allyl, $CH_2$-$CH=CH$-$R^8$ und b eine Zahl 1, 2, 3 oder 4;

$R^3$ Wasserstoff, $C_1$-$C_{12}$-Alkyl, Formyl, Acetyl, halogensubstituiertes Acetyl,

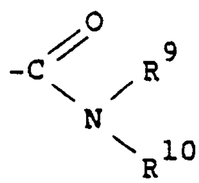 , 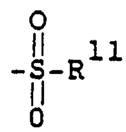 ;

$R^4$ Wasserstoff, $C_1$-$C_{12}$-Alkyl, $C_3$-$C_{12}$-Alkenyl, $C_3$-$C_{12}$-Alkinyl;
$-CH=CHR^{12}$, $-C{\equiv}CR^{13}$, $-CH_2$-O-$R^{14}$,

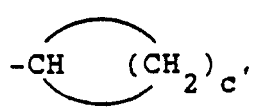 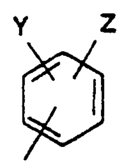 ,

Aryloxy, Aralkoxy, Alkylthio, Arylthio, Aralkylthio, Aryloxycarbonyl, Heteroaryl und substituiertes Heteroaryl, wie

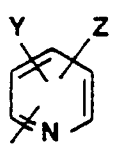 , 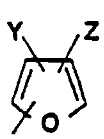 , 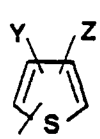 , 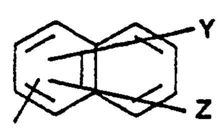 , 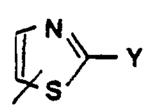 ,

und

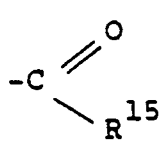 , 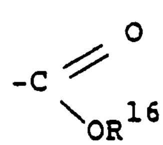 ,

worin
Y Wasserstoff, Halogen, Niederalkyl, Alkoxy, OH, $NH_2$, $NO_2$,
Z Wasserstoff, Halogen, Niederalkyl, Alkoxy, OH, $NH_2$, $NO_2$ und
c eine Zahl 1, 2, 3, 4 oder 5;

$R^6$ 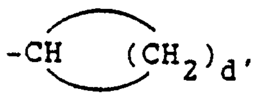 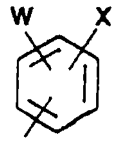 ,

Aryloxy, Aralkoxy, Arylthio, Alkylthio, Aralkylthio, Aryloxycarbonyl, Heteroaryl und substituiertes Heteroaryl, wie

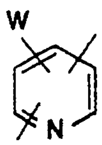 , 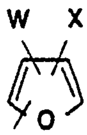 , 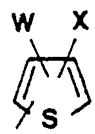 , 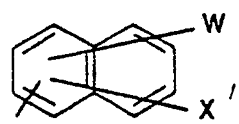 , 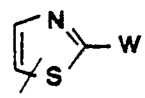 ,

und

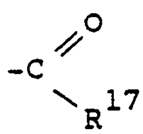 , 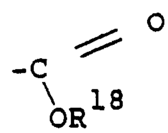 ,

worin
W Wasserstoff, Halogen, Niederalkyl, Alkoxy, OH, $NH_2$, $NO_2$,
X Wasserstoff, Halogen, Niederalkyl, Alkoxy, OH, $NH_2$, $NO_2$ und
d eine Zahl 1, 2, 3, 4 oder 5;
$R^7$ $-CH=CHR^{19}$, $-C{\equiv}CR^{20}$, $-CH_2-O-R^{21}$,

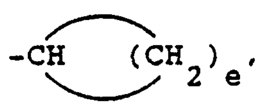 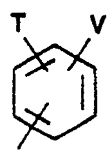 ,

Aryloxy, Aralkoxy, Alkylthio, Arylthio, Aralkylthio, Aryloxycarbonyl, Heteroaryl und substituiertes Heteroaryl, wie

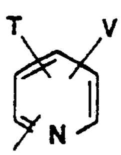 , 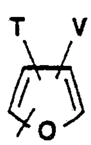 , 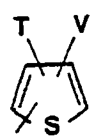 , 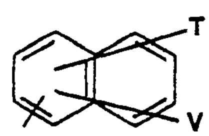 , 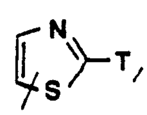 ,

und

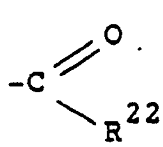 , 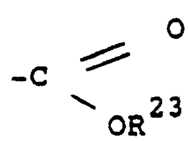 ,

worin
T Wasserstoff, Halogen, Niederalkyl, Alkoxy, OH, $NH_2$, $NO_2$,

V Wasserstoff, Halogen, Niederalkyl, Alkoxy, OH, $NH_2$, $NO_2$ und
e eine Zahl 1, 2, 3, 4 oder 5;

$R^8$ 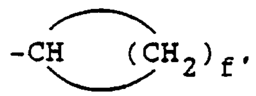 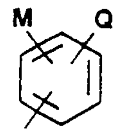 ,

Aryloxy, Aralkoxy, Arylthio , Alkylthio, Aralkylthio, Aryloxycarbonyl, Heteroaryl und substituiertes Heteroaryl, wie

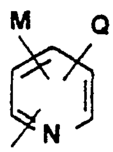 , 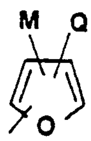 , 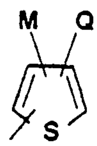 , 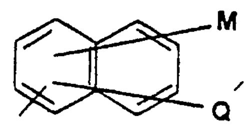 , 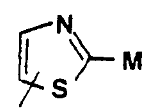

und

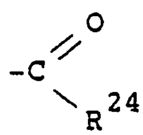 , 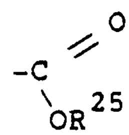 ,

worin
M Wasserstoff, Halogen, Niederalkyl, Alkoxy, OH, $NH_2$, $NO_2$,
Q Wasserstoff, Halogen, Niederalkyl, Alkoxy, OH, $NH_2$, $NO_2$ und
f eine Zahl 1, 2, 3, 4 oder 5;
$R^9$ Wasserstoff, Alkyl;
$R^{10}$ Wasserstoff, Alkyl;
$R^9$ und $R^{10}$ auch zusammen mit dem Stickstoffatom einen Heterocyclus, der noch ein weiteres Heteroatom - wie Stickstoff, Sauerstoff oder Schwefel - enthalten kann - z.B. einen Morpholin-, Piperidin- oder Piperazinring -bilden können;
$R^{11}$ Alkyl, Aralkyl;
$R^{12}$ Wasserstoff, Alkyl, Aryl;
$R^{13}$ Wasserstoff, Alkyl;
$R^{14}$ Wasserstoff, Alkyl;
$R^{15}$ Alkyl, Aryl;
$R^{16}$ Alkyl, Aryl;
$R^{17}$ Alkyl, Aryl;
$R^{18}$ Alkyl, Aryl;
$R^{19}$ Wasserstoff, Alkyl, Aryl;
$R^{20}$ Wasserstoff, Alkyl, Aryl;
$R^{21}$ Wasserstoff, Alkyl;
$R^{22}$ Alkyl, Aryl;
$R^{23}$ Alkyl, Aryl;
$R^{24}$ Alkyl, Aryl;
$R^{25}$ Alkyl, Aryl;
bedeuten, wobei $R^1$, $R^2$, $R^3$ und $R^4$ nicht alle zusammen Wasserstoff und - im Falle $R^3$ und $R^4$ beide Wasserstoff oder $R^3$ Acetyl und $R^4$ Wasserstoff bedeuten - $R^1$ und $R^2$ nicht beide Methyl oder nicht beide Propyl bedeuten dürfen.

Bevorzugt sind Verbindungen der allgemeinen Formel I, worin
$R^1$ Wasserstoff, $C_1$-$C_6$-Alkyl, $(CH_2)_2$-$R^5$, $CH_2$-$CH=CH$-$R^6$, Alkoxycarbonyl und
a eine Zahl 1, 2, 3 oder 4;
$R^2$ Wasserstoff, $C_1$-$C_6$-Alkyl, $(CH_2)_b$-$R^7$, Allyl, $CH_2$-$CH=CH$-$R^8$ und
b eine Zahl 1, 2, 3 oder 4;
$R^3$ Wasserstoff, $C_1$-$C_6$-Alkyl, Formyl, Acetyl, halogensubstituiertes Acetyl,

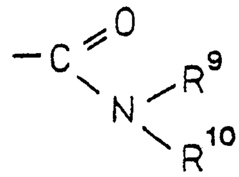 / 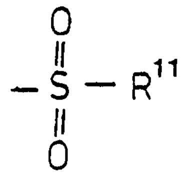 ;

$R^4$ Wasserstoff, $C_1$-$C_6$-Alkyl, $C_3$-$C_6$-Alkenyl, $C_3$-$C_6$-Alkinyl;
$R^5$, $R^6$, $R^7$ und $R^8$ die oben angegebene Bedeutung haben;
$R^9$ und $R^{10}$ Wasserstoff oder Niederalkyl bedeuten, wobei $R^9$ und $R^{10}$ auch zusammen mit dem Stickstoffatom einen 5- oder 6- gliedrigen Heterocyclus - wie z.B. einen Morpholin-, Piperidin- oder Piperazinring - bilden können, in dem ein Kohlenstoffatom durch ein Heteroatom - wie z. B. Stickstoff, Sauerstoff oder Schwefel - ersetzt sein kann;
$R^{11}$ Niederalkyl oder Aralkyl bedeutet.

Besonders bevorzugt sind Verbindungen der allgemeinen Formel I, worin
$R^1$ Wasserstoff, $C_1$-$C_4$-Alkyl, Allyl, tert.-Butoxycarbonyl;
$R^2$ Wasserstoff, $C_1$-$C_4$-Alkyl, $(CH_2)_b$-$R^7$, Allyl, $CH_2$-$CH=CH$-$R^8$,
b eine Zahl 1, 2, 3 oder 4;
$R^3$ Wasserstoff, Formyl, Acetyl, Trifluoracetyl,

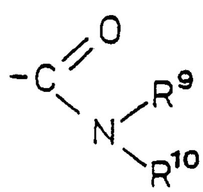 / 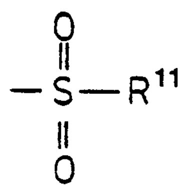 ;

$R^4$ Wasserstoff, $C_1$-$C_4$-Alkyl;
$R^7$ $-CH=CHR^{12}$, $-C{\equiv}CR^{13}$, Cyclopropyl, Phenyl,

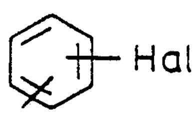 , 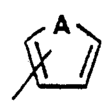 ;

worin Hal Chlor oder Brom und A Sauerstoff oder Schwefel;

$R^8$ 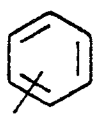 , 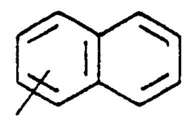 ;

$R^9$ und $R^{10}$ Wasserstoff, Niederalkyl;
$R^{11}$ Niederalkyl;
$R^{12}$ Wasserstoff, Niederalkyl, Phenyl;
$R^{13}$ Wasserstoff oder Niederalkyl bedeutet.

Die erfindungsgemäßen 2,7-Diamino-1,2,3,4-tetrahydronaphthaline (2,7-Diaminotetraline) weisen mindestens ein C-Atom mit einem Asymmetriezentrum auf und können je nach Substitutionsmuster auch mehrere Asymmetriezentren besitzen und können daher in verschiedenen stereochemischen Formen existieren.

Als Beispiele seien folgende Isomeren der substituierten 2,7-Diaminotetraline der allgemeinen Formel II und III genannt:

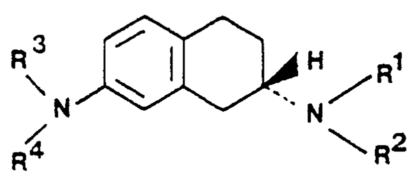

(II)

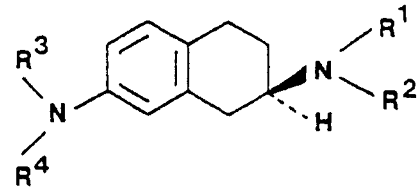

(III)

Gegenstand der Erfindung sind die einzelnen Isomeren, deren Mischungen sowie die entsprechenden physiologisch geeigneten Säureadditionssalze mit anorganischen oder organischen Säuren. Bevorzugt sind beispielsweise Salze mit Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Milchsäure, Malonsäure, Bernsteinsäure, Maleinsäure, Fumarsäure, Äpfelsäure, Weinsäure, Zitronensäure oder Benzoesäure.

Soweit nicht im einzelnen abweichende Angaben gemacht werden, werden die allgemeinen Definitionen im folgenden Sinn gebraucht:

Alkyl steht im allgemeinen für einen unverzweigten oder verzweigten Kohlenwasserstoffrest mit 1 bis 12 Kohlenstoffatomen, der gegebenenfalls mit einem Halogenatom oder mehreren Halogenatomen substituiert sein kann - vorzugsweise Fluor - die untereinander gleich oder verschieden sein können, wobei Niederalkylreste bevorzugt sind. Niederalkyl steht im allgemeinen für einen verzweigten oder unverzweigten Kohlenwasserstoffrest mit 1 bis etwa 6 Kohlenstoffatomen. Als Beispiel seien Methyl, Ethyl, Propyl, Isopropyl, Butyl, Isobutyl, Pentyl, Isopentyl, Neopentyl, Hexyl, Isohexyl, Heptyl, Isoheptyl, Octyl sowie Isooctyl genannt.

Alkenyl steht im allgemeinen für einen geradkettigen oder verzweigten Kohlenwasserstoffrest mit 3 bis 12 Kohlenstoffatomen und mit einer oder mehreren, bevorzugt mit einer oder zwei Doppelbindung(en), der gegebenenfalls mit einem Halogenatom oder mehreren Halogenatomen - vorzugsweise Fluor - substituiert sein kann, die untereinander gleich oder verschieden sein können. Bevorzugt ist ein Niederalkenylrest mit 3 bis etwa 6 Kohlenstoffatomen und einer oder zwei Doppelbindung(en). Besonders bevorzugt ist ein Alkenylrest mit 3 oder 4 Kohlenstoffatomen und einer Doppelbindung. Als Beispiele seien Allyl, Propenyl, Isopropenyl, Pentenyl, Isopentenyl, Hexenyl, Isohexenyl, Heptenyl, Isoheptenyl, Octenyl und Isooctenyl genannt.

Alkinyl steht im allgemeinen für einen geradkettigen oder verzweigten Kohlenwasserstoffrest mit 3 bis 12 Kohlenstoffatomen und mit einer oder mehreren, bevorzugt mit einer oder zwei Dreifachbindung(en). Bevorzugt ist ein Niederalkinylrest mit 3 bis etwa 6 Kohlenstoffatomen und einer oder zwei Dreifachbindung(en), der gegebenenfalls mit einem Halogenatom oder mehreren Halogenatomen - vorzugsweise Fluor - substituiert sein kann, die untereinander gleich oder verschieden sein können. Besonders bevorzugt ist ein Alkinylrest mit 3 oder 4 Kohlenstoffatomen und einer Dreifachbindung. Als Beispiele seien Propargyl und Butinyl(2) genannt.

Cycloalkyl steht im allgemeinen für einen gesättigten oder ungesättigten cyclischen Kohlenwasserstoffrest mit 5 bis 9 Kohlenstoffatomen, der gegebenenfalls mit einem Halogenatom oder mehreren Halogenatomen - vorzugsweise Fluor - substituiert sein kann, die untereinander gleich oder verschieden sein können. Bevorzugt sind cyclische Kohlenwasserstoffe mit 3 bis 6 Kohlenstoffatomen. Als Beispiele seien Cyclopropyl, Cyclobutyl, Cyclopentyl, Cyclopentenyl, Cyclohexyl, Cyclohexenyl, Cycloheptyl, Cycloheptenyl, Cycloheptadienyl, Cyclooctyl, Cyclooctenyl, Cyclooctadienyl und Cyclononinyl genannt.

Aryl steht im allgemeinen für einen aromatischen Rest mit 6 bis 10 Kohlenstoffatomen - auch in Zusammensetzungen, wobei der Aromat mit einer oder mehreren Niederalkylgruppe(n), Alkoxygruppe(n), Nitrogruppe(n), Aminogruppe(n) und/oder einem oder mehreren Halogenatom(en) - untereinander gleich oder verschieden - substituiert sein kann. Bevorzugte Arylreste sind Phenyl, Naphthyl und Biphenyl.

Aralkyl steht im allgemeinen für einen über eine Alkylenkette gebundenen Arylrest mit 7 bis 14 Kohlenstoffatomen, wobei der Aromat mit einer oder mehreren Niederalkylgruppe(n), Alkoxygruppe(n), Nitrogruppe(n), Aminogruppe(n) und/oder einem oder mehreren Halogenatom(en) - untereinander gleich oder verschieden - substituiert sein kann. Bevorzugt werden Aralkylreste mit 1 bis 6 Kohlenstoffatomen im aliphatischen Teil und 6 bis 10 Kohlenstoffatomen im aromatischen Teil. Als bevorzugte Aralkylreste seien genannt: Benzyl, Naphthylmethyl, Phenetyl und Phenylpropyl.

Alkoxy steht im allgemeinen für einen über ein Sauerstoffatom gebundenen geradkettigen oder

verzweigten Kohlenwasserstoffrest mit 1 bis 12 Kohlenstoffatomen. Bevorzugt ist Niederalkoxyrest mit 1 bis etwa 6 Kohlenstoffatomen. Besonders bevorzugt ist ein Alkoxyrest mit 1 bis 4 Kohlenstoffatomen. Als Beispiele seien Methoxy, Ethoxy, Propoxy, Isopropoxy, Butoxy, Isobutoxy, tert.-Butoxy, Pentoxy, Isopentoxy, Hexoxy, Isohexoxy, Heptoxy, Isoheptoxy, Octoxy oder Isooctoxy genannt.

Aryloxy steht im allgemeinen für einen aromatischen Rest mit 6 bis etwa 12 Kohlenstoffatomen, der über ein Sauerstoffatom gebunden ist und gegebenenfalls mit einer oder mehreren Niederlakylgruppe(n), Alkoxygruppe(n), Nitrogruppe(n), Aminogruppe(n) und/oder Halogenatom(en) - untereinander gleich oder verschieden - substituiert sein kann. Bevorzugte Aryloxyreste sind Phenoxy oder Naphthyloxy.

Aralkoxy steht im allgemeinen für einen Aralkylrest mit 7 bis 14 Kohlenstoffatomen, wobei die Alkylenkette über ein Sauerstoffatom gebunden ist. Bevorzugt werden Aralkoxyreste mit 1 bis 6 Kohlenstoffatomen im aliphatischen Teil und 6 bis 10 Kohlenstoffatomen im aromatischen Teil. Beispielsweise seien folgende Aralkoxyreste genannt: Benzyloxy, Naphthmethyloxy, Phenethoxy und Phenylpropoxy.

Alkylthio steht im allgemeinen für einen über ein Schwefelatom gebundenen geradkettigen oder verzweigten Kohlenwasserstoffrest mit 1 bis 12 Kohlenstoffatomen. Bevorzugt ist Niederalkylthio mit 1 bis 6 Kohlenstoffatomen. Besonders bevorzugt ist ein Alkylthiorest mit 1 bis 4 Kohlenstoffatomen. Als Beispiele seien Methylthio, Ethylthio, Propylthio, Isopropylthio, Butylthio, Isobutylthio, Hexylthio, Isohexylthio, Heptylthio, Isoheptylthio, Octylthio und Isooctylthio genannt.

Arylthio steht im allgemeinen für einen aromatischen Rest mit 6 bis etwa 12 Kohlenstoffatomen, der über ein Schwefelatom gebunden ist, und gegebenenfalls mit einer oder mehreren Niederalkylgruppe(n), Alkoxygruppe(n), Nitrogruppe(n), Aminogruppe(n) und/oder Halogenatom(en) - untereinander gleich oder verschieden - substituiert sein kann. Als Beispiele seien der Phenylthio- und der Naphthylthiorest genannt, wobei der Phenylthiorest bevorzugt wird.

Aralkylthio steht im allgemeinen für einen Aralkylrest mit 7 bis 14 Kohlenstoffatomen, wobei die Alkylenkette über ein Schwefelatom gebunden ist. Bevorzugt werden Aralkylthioreste mit 1 bis 6 Kohlenstoffatomen im aliphatischen Teil und mit 6 bis 12 Kohlenstoffatomen im aromatischen Teil, wobei sowohl der aliphatische als auch der aromatische Teil mit einer oder mehreren Niederalkylgruppe(n), Alkoxygruppe(n), Nitrogruppe(n), Aminogruppe(n) und/oder Halogenatom(en) - untereinander gleich oder verschieden - substituiert sein kann. Als Beispiele seien der Benzylthio- und der Phenethylthiorest genannt.

Acyl steht im allgemeinen für Benzoyl oder für einen Alkylcarbonylrest - wie geradkettiges oder verzweigtes Niederalkyl mit 1 bis etwa 6 Kohlenstoffatomen, die über eine Carbonylgruppe gebunden sind, wobei der Alkylrest gegebenenfalls mit einem oder mehreren Halogenatom(en), die untereinander gleich oder verschieden sein können, substituiert sein kann. Bevorzugt sind Alkylreste mit bis zu 4 Kohlenstoffatomen. Als Beispiele seien genannt: Benzoyl, Acetyl, Ethylcarbonyl, Propylcarbonyl, Isopropylcarbonyl, Butylcarbonyl sowie Isobutylcarbonyl.

Alkoxycarbonyl kann beispielsweise durch die Formel

$$-\underset{\underset{O}{\|}}{C}-O\text{-Alkyl}$$

dargestellt werden.

Alkyl steht hierbei für einen geradkettigen oder verzweigten Kohlenwasserstoffrest mit 1 bis 12 Kohlenstoffatomen. Bevorzugt wird ein Niederalkoxycarbonylrest mit 1 bis 6 Kohlenstoffatomen. Besonders bevorzugt wird ein Alkoxycarbonylrest mit 1 bis 4 Kohlenstoffatomen im Alkylrest. Als Beispiele seien folgende Alkoxycarbonylreste genannt: Methoxycarbonyl, Ethoxycarbonyl, Propoxycarbonyl, Isopropoxycarbonyl, Butoxycarbonyl oder Isobutoxycarbonyl.

Aryloxycarbonyl kann durch die Formel

$$-\underset{\underset{O}{\|}}{C}-O\text{-Aryl}$$

dargestellt werden.

Aryl steht hierbei im allgemeinen für einen aromatischen Rest mit 6 bis 12 Kohlenstoffatomen - zum Beispiel: Phenoxycarbonyl, Naphthyloxycarbonyl.

Aralkoxycarbonyl kann durch die Formel

$$-\underset{\underset{O}{\|}}{C}-O\text{-Aralkyl}$$

dargestellt werden.

Aralkyl steht hierbei für einen über eine Alkylenkette gebundenen Arylrest mit 7 bis 14 Kohlenstoffatomen ; bevorzugt werden Aralkylreste mit 1 bis 6 Kohlenstoffatomen im aliphatischen Teil und 6 bis 12 Kohlenstoffatomen im aromatischen Teil. Als Beispiele für Aralkoxycarbonylreste seien Benzyloxycarbonyl Naphthylmethyloxycarbonyl genannt.

Heteroaryl im Rahmen der oben angegebenen Definition steht im allgemeinen für einen 5- bis 6-

gliedrigen Ring, der als Heteroatome Sauerstoff, Schwefel und/oder Stickstoff enthalten kann und an den ein weiterer aromatischer Ring ankondensiert sein kann. Bevorzugt sind 5- und 6-gliedrige aromatische Ringe, die einen Sauerstoff, einen Schwefel und/oder bis zu zwei Stickstoffatomen enthalten und die gegebenenfalls benzokondensiert sind. Als besondere Heteroarylreste seien beispielsweise Thienyl, Furyl, Pyridyl, Pyrimidyl, Pyrazinyl, Pyridazinyl, Chinolyl, Isochinolyl, Chinazolyl, Chinoxalyl, Thiazolyl, Benzothiazolyl, Isothiazolyl, Oxazolyl, Benzoxazolyl, Isoxazolyl, Imidazolyl, Benzimidazolyl, Pyrazolyl und Indolyl genannt.

Bevorzugte Bedeutung für $R^1$ ist Wasserstoff, Methyl, Ethyl, Propyl, Butyl oder Allyl.

Bevorzugte Bedeutung für $R^2$ ist Propyl, Butyl, Cyclopropylmethyl, Allyl, Propargyl, Phenethyl, 3-Phenylpropyl, 4-Phenylbutyl, 3-Phenylpropargyl, 2-(2-Thienyl)ethyl, 2-(3-Thienyl)ethyl, 2-(4-Chlorphenyl)-ethyl und 3-(4-Chlorphenyl)propyl.

Bevorzugte Bedeutung für $R^3$ ist Formyl, Acetyl oder halogensubstituiertes Acetyl.

Bevorzugte Bedeutung für $R^4$ ist Wasserstoff.

Halogen bedeutet - sofern nicht anders angegeben -Chlor und Brom, daneben auch Fluor in zweiter Linie Jod.

Beispielsweise seien genannt:

7-Acetamido-2-methylamino-1,2,3,4-tetrahydronaphthalin
7-Acetamido-2-ethylamino-1,2,3,4-tetrahydronaphthalin
7-Acetamido-2-propylamino-1,2,3,4-tetrahydronaphthalin
7-Acetamido-2-[2-(2-thienyl)ethyl]amino-1,2,3,4-tetrahydronaphthalin
7-Acetamido-2-[2-(3-thienyl)ethyl]amino-1,2,3,4-tetrahydronaphthalin
7-Acetamido-2-N,N-dibutylamino-1,2,3,4-tetrahydronaphthalin
7-Acetamido-2-(N-butyl-N-propyl)amino-1,2,3,4-tetrahydronaphthalin
7-Acetamido-2-[N-2-(2-thienyl)ethyl-N-propyl]amino-1,2,3,4-tetrahydronaphthalin
7-Acetamido-2-[N-2-(3-thienyl)ethyl-N-propyl]amino-1,2,3,4-tetrahydronaphthalin
7-Acetamido-2-(N-benzyl-N-propyl)amino-1,2,3,4-tetrahydronaphthalin
7-Acetamido-2-(N-phenethyl-N-propyl)amino-1,2,3,4-tetrahydronaphthalin
7-Acetamido-2-[N-(3-phenylpropyl)-N-propyl]amino-1,2,3,4-tetrahydronaphthalin
7-Acetamido-2-(N-allyl-N-propyl)amino-1,2,3,4-tetrahydronaphthalin
7-Acetamido-2-(3-phenylallyl)amino-1,2,3,4-tetrahydronaphthalin
7-Acetamido-2-[N-(3-phenylallyl)-N-propyl]amino-1,2,3,4-tetrahydronaphthalin
7-Acetamido-2-N,N-diallylamino-1,2,3,4-tetrahydronaphthalin
7-Acetamido-2-(N-propargyl-N-propyl)amino-1,2,3,4-tetrahydronaphthalin
7-Acetamido-2-[N-(4-phenylbutyl)-N-propyl]amino-1,2,3,4-tetrahydronaphthalin
7-Acetamido-2-[N-2-(4-chlorphenyl)ethyl-N-propyl]amino-1,2,3,4-tetrahydronaphthalin
7-Acetamido-2-(N-isopropyl-N-propyl)amino-1,2,3,4-tetrahydronaphthalin
7-Acetamido-2-(N-cyclopropylmethyl-N-propyl)amino-1,2,3,4-tetrahydronaphthalin
7-Acetamido-2-N,N-di(3-phenylpropyl)amino-1,2,3,4-tetrahydronaphthalin
7-Acetamido-2-{N-[3-(4-chlorphenyl)propyl]-N-propyl}amino-1,2,3,4-tetrahydronaphthalin
7-Acetamido-2-(N-methyl-N-propyl)amino-1,2,3,4-tetrahydronaphthalin
(-)-7-Acetamido-2-(N-butyl-N-propyl)amino-1,2,3,4-tetrahydronaphthalin
(+)-7-Acetamido-2-(N-butyl-N-propyl)amino-1,2,3,4-tetrahydronaphthalin
(-)-7-Acetamido-2-(N-pentyl-N-propyl)amino-1,2,3,4-tetrahydronaphthalin
(+)-7-Acetamido-2-(N-pentyl-N-propyl)amino-1,2,3,4-tetrahydronaphthalin
(-)-7-Acetamido-2-(N-methyl-N-phenethyl)amino-1,2,3,4-tetrahydronaphthalin
(+)-7-Acetamido-2-(N-methyl-N-phenethyl)amino-1,2,3,4-tetrahydronaphthalin
(-)-7-Acetamido-2-(N-phenethyl-N-propyl)amino-1,2,3,4-tetrahydronaphthalin
(+)-7-Acetamido-2-(N-phenethyl-N-propyl)amino-1,2,3,4-tetrahydronaphthalin
(-)-7-Acetamido-2-[N-(3-phenylpropyl)-N-propyl]amino-1,2,3,4-tetrahydronaphthalin
(+)-7-Acetamido-2-[N-(3-phenylpropyl)-N-propyl]amino-1,2,3,4-tetrahydronaphthalin
(-)-7-Acetamido-2-[N-(3-phenylallyl)-N-propyl]amino-1,2,3,4-tetrahydronaphthalin
(+)-7-Acetamido-2-[N-(3-phenylallyl)-N-propyl]amino-1,2,3,4-tetrahydronaphthalin
(-)-7-Acetamido-2-[N-(2-methoxyethyl)-N-propyl]amino-1,2,3,4-tetrahydronaphthalin
(+)-7-Acetamido-2-[N-(2-methoxyethyl)-N-propyl]amino-1,2,3,4-tetrahydronaphthalin
(-)-7-Acetamido-2-[N-(2-phenoxyethyl)-N-propyl]amino-1,2,3,4-tetrahydronaphthalin
(+)-7-Acetamido-2-[N-(2-phenoxyethyl)-N-propyl]amino-1,2,3,4-tetrahydronaphthalin
(-)-7-Acetamido-2-[N-(2-benzyloxyethyl)-N-propyl]amino-1,2,3,4-tetrahydronaphthalin
(+)-7-Acetamido-2-[N-(2-benzyloxyethyl)-N-propyl]amino-1,2,3,4-tetrahydronaphthalin
(-)-7-Acetamido-2-[N-(3-methoxypropyl)-N-propyl]amino-1,2,3,4-tetrahydronaphthalin

(+)-7-Acetamido-2-[N-(3-methoxypropyl)-N-propyl]amino-1,2,3,4-tetrahydronaphthalin
(-)-7-Acetamido-2-[N-(2-methylthioethyl)-N-propyl]amino-1,2,3,4-tetrahydronaphthalin
(+)-7-Acetamido-2-[N-(2-methylthioethyl)-N-propyl]amino-1,2,3,4-tetrahydronaphthalin
(-)-7-Acetamido-2-[N-(2-phenylthioethyl)-N-propyl]amino-1,2,3,4-tetrahydronaphthalin
(+)-7-Acetamido-2-[N-(2-phenylthioethyl)-N-propyl]amino-1,2,3,4-tetrahydronaphthalin
(-)-7-Acetamido-2-[N-(3-methylthiopropyl)-N-propyl]amino-1,2,3,4-tetrahydronaphthalin
(+)-7-Acetamido-2-[N-(3-methylthiopropyl)-N-propyl]amino-1,2,3,4-tetrahydronaphthalin
(-)-7-Acetamido-2-[N-(but-3-enyl)-N-propyl]amino-1,2,3,4-tetrahydronaphthalin
(+)-7-Acetamido-2-[N-(but-3-enyl)-N-propyl]amino-1,2,3,4-tetrahydronaphthalin
(-)-7-Acetamido-2-[N-(2-methoxycarbonylethyl)-N-propyl]amino-1,2,3,4-tetrahydronaphthalin
(+)-7-Acetamido-2-[N-(2-methoxycarbonylethyl)-N-propyl]amino-1,2,3,4-tetrahydronaphthalin
(-)-7-Acetamido-2-[N-(2-phenoxycarbonylethyl)-N-propyl]amino-1,2,3,4-tetrahydronaphthalin
(+)-7-Acetamido-2-[N-(2-phenoxycarbonylethyl)-N-propyl]amino-1,2,3,4-tetrahydronaphthalin
(-)-7-Acetamido-2-[N-(2-hydroxycarbonylethyl)-N-propyl]amino-1,2,3,4-tetrahydronaphthalin
(+)-7-Acetamido-2-[N-(2-hydroxycarbonylethyl)-N-propyl]amino-1,2,3,4-tetrahydronaphthalin
(-)-7-Acetamido-2-[N-(3-oxo-butyl)-N-propyl]amino-1,2,3,4-tetrahydronaphthalin
(+)-7-Acetamido-2-[N-(3-oxo-butyl)-N-propyl]amino-1,2,3,4-tetrahydronaphthalin
(-)-7-Acetamido-2-{N-[2-(2-furyl)ethyl]-N-propyl}amino-1,2,3,4-tetrahydronaphthalin
(+)-7-Acetamido-2-{N-[2-(2-furyl)ethyl]-N-propyl}amino-1,2,3,4-tetrahydronaphthalin
(-)-7-Acetamido-2-{N-[2-(3-furyl)ethyl]-N-propyl}amino-1,2,3,4-tetrahydronaphthalin
(+)-7-Acetamido-2-{N-[2-(3-furyl)ethyl]-N-propyl}amino-1,2,3,4-tetrahydronaphthalin
(-)-7-Acetamido-2-{N-propyl-N-[2-(2-pyridyl)ethyl]}amino-1,2,3,4-tetrahydronaphthalin
(+)-7-Acetamido-2-{N-propyl-N-[2-(2-pyridyl)ethyl]}amino-1,2,3,4-tetrahydronaphthalin
(-)-7-Acetamido-2-{N-propyl-N-[2-(3-pyridyl)ethyl]}amino-1,2,3,4-tetrahydronaphthalin
(+)-7-Acetamido-2-{N-propyl-N-[2-(3-pyridyl)ethyl]}amino-1,2,3,4-tetrahydronaphthalin
(-)-7-Acetamido-2-{N-propyl-N-[2-(4-pyridyl)ethyl]}amino-1,2,3,4-tetrahydronaphthalin
(+)-7-Acetamido-2-{N-propyl-N-[2-(4-pyridyl)ethyl]}amino-1,2,3,4-tetrahydronaphthalin
(-)-7-Acetamido-2-{N-[2-(4-methoxyphenyl)ethyl]-N-propyl}amino-1,2,3,4-tetrahydronaphthalin
(+)-7-Acetamido-2-{N-[2-(4-methoxyphenyl)ethyl]-N-propyl}amino-1,2,3,4-tetrahydronaphthalin
(-)-7-Acetamido-2-{N-[2-(4-methylphenyl)ethyl]-N-propyl}amino-1,2,3,4-tetrahydronaphthalin
(+)-7-Acetamido-2-{N-[2-(4-methylphenyl)ethyl]-N-propyl}amino-1,2,3,4-tetrahydronaphthalin
(-)-7-Acetamido-2-{N-[2-(4-hydroxyphenyl)ethyl]-N-propyl}amino-1,2,3,4-tetrahydronaphthalin
(+)-7-Acetamido-2-{N-[2-(4-hydroxyphenyl)ethyl]-N-propyl}amino-1,2,3,4-tetrahydronaphthalin
(-)-7-Acetamido-2-{N-[2-(3,4-dimethoxyphenyl)ethyl]-N-propyl}amino-1,2,3,4-tetrahydronaphthalin
(+)-7-Acetamido-2-{N-[2-(3,4-dimethoxyphenyl)ethyl]-N-propyl}amino-1,2,3,4-tetrahydronaphthalin
(-)-7-Acetamido-2-{N-[2-(3-chlorphenyl)ethyl]-N-propyl}amino-1,2,3,4-tetrahydronaphthalin
(+)-7-Acetamido-2-{N-[2-(3-chlorphenyl)ethyl]-N-propyl}amino-1,2,3,4-tetrahydronaphthalin
(-)-7-Acetamido-2-{N-[2-(3,4-dichlorphenyl)ethyl]-N-propyl}amino-1,2,3,4-tetrahydronaphthalin
(+)-7-Acetamido-2-{N-[2-(3,4-dichlorphenyl)ethyl]-N-propyl}amino-1,2,3,4-tetrahydronaphthalin
(-)-7-Acetamido-2-{N-[2-(3,5-dichlorphenyl)ethyl]-N-propyl}amino-1,2,3,4-tetrahydronaphthalin
(+)-7-Acetamido-2-{N-[2-(3,5-dichlorphenyl)ethyl]-N-propyl}amino-1,2,3,4-tetrahydronaphthalin
(-)-7-Acetamido-2-{N-[2-(1-naphthyl)ethyl]-N-propyl}amino-1,2,3,4-tetrahydronaphthalin
(+)-7-Acetamido-2-{N-[2-(1-naphthyl)ethyl]-N-propyl}amino-1,2,3,4-tetrahydronaphthalin
(-)-7-Acetamido-2-{N-[2-(2-naphthyl)ethyl]-N-propyl}amino-1,2,3,4-tetrahydronaphthalin
(+)-7-Acetamido-2-{N-[2-(2-naphthyl)ethyl]-N-propyl}amino-1,2,3,4-tetrahydronaphthalin
(-)-7-Acetamido-2-[N-[2-phenylpropyl)-N-propyl]amino-1,2,3,4-tetrahydronaphthalin
(+)-7-Acetamido-2-[N-[2-phenylpropyl)-N-propyl]amino-1,2,3,4-tetrahydronaphthalin
(-)-7-Acetamido-2-[N-[2-cyclohexylethyl)-N-propyl]amino-1,2,3,4-tetrahydronaphthalin
(+)-7-Acetamido-2-[N-[2-cyclohexylethyl)-N-propyl]amino-1,2,3,4-tetrahydronaphthalin
(-)-7-Acetamido-2-{N-propyl-N-[3-(2-pyridyl)allyl]}amino-1,2,3,4-tetrahydronaphthalin
(+)-7-Acetamido-2-{N-propyl-N-[3-(2-pyridyl)allyl]}amino-1,2,3,4-tetrahydronaphthalin
7-Amino-2-methylamino-1,2,3,4-tetrahydronaphthalin
7-Amino-2-ethylamino-1,2,3,4-tetrahydronaphthalin
7-Amino-2-propylamino-1,2,3,4-tetrahydronaphthalin
7-Amino-2-benzylamino-1,2,3,4-tetrahydronaphthalin
7-Amino-2-[2-(2-thienyl)ethyl]amino-1,2,3,4-tetrahydronaphthalin
7-Amino-2-[2-(3-thienyl)ethyl]amino-1,2,3,4-tetrahydronaphthalin
7-Amino-2-N,N-dimethylamino-1,2,3,4-tetrahydronaphthalin

7-Amino-2-N,N-diethylamino-1,2,3,4-tetrahydronaphthalin
7-Amino-2-N,N-dipropylamino-1,2,3,4-tetrahydronaphthalin
7-Amino-2-N,N-dibutylamino-1,2,3,4-tetrahydronaphthalin
7-Amino-2-(N-methyl-N-propyl)amino-1,2,3,4-tetrahydronaphthalin
7-Amino-2-(N-butyl-N-propyl)amino-1,2,3,4-tetrahydronaphthalin
7-Amino-2-(N-benzyl-N-methyl)amino-1,2,3,4-tetrahydronaphthalin
7-Amino-2-(N-benzyl-N-ethyl)amino-1,2,3,4-tetrahydronaphthalin
7-Amino-2-(N-benzyl-N-propyl)amino-1,2,3,4-tetrahydronaphthalin
7-Amino-2-(N-phenethyl-N-propyl)amino-1,2,3,4-tetrahydronaphthalin
7-Amino-2-(N-methyl-N-phenethyl)amino-1,2,3,4-tetrahydronaphthalin
7-Amino-2-[N-(3-phenylpropyl)-N-propyl]amino-1,2,3,4-tetrahydronaph thalin
2-(N-Allyl-N-methyl)amino-7-amino-1,2,3,4-tetrahydronaphthalin
2-(N-Allyl-N-ethyl)amino-7-amino-1,2,3,4-tetrahydronaphthalin
2-(N-Allyl-N-propyl)amino-7-amino-1,2,3,4-tetrahydronaphthalin
7-Amino-2-N,N-diallyl-1,2,3,4-tetrahydronaphthalin
7-Amino-2-(N-propargyl-N-propyl)amino-1,2,3,4-tetrahydronaphthalin
7-Amino-2-N,N-dipropargylamino-1,2,3,4-tetrahydronaphthalin
7-Amino-2-[N-(4-phenylbutyl)-N-propyl]amino-1,2,3,4-tetrahydronaphthalin
7-Amino-2-{N-[2-(4-chlorphenyl)ethyl]-N-propyl}amino-1,2,3,4-tetrahydronaphthalin
7-Amino-2-[N-ethyl-N-(2-pyridyl)methyl]amino-1,2,3,4-tetrahydronaphthalin
7-Amino-2-{N-propyl-N-[(2-thienyl)methyl]}amino-1,2,3,4-tetrahydronaphthalin
7-Amino-2-{N-propyl-N-[(3-thienyl)methyl]}amino-1,2,3,4-tetrahydronaphthalin
7-Amino-2-{N-propyl-N-[2-(2-thienyl)ethyl]}amino-1,2,3,4-tetrahydronaphthalin
7-Amino-2-{N-propyl-N-[2-(3-thienyl)ethyl]}amino-1,2,3,4-tetrahydronaphthalin
7-Amino-2-(N-cyclopropylmethyl-N-propyl)amino-1,2,3,4-tetrahy dronaphthalin
7-Amino-2-[N-(3-phenyl)allyl-N-propyl]amino-1,2,3,4-tetrahydronaphthalin
7-Amino-2-[N-(3-phenylpropargyl)-N-propyl]amino-1,2,3,4-tetrahydronaphthalin
7-Amino-2-N,N-di(3-phenylpropyl)amino-1,2,3,4-tetrahydronaphthalin
(-)-7-Amino-2-(N-butyl-N-propyl)amino-1,2,3,4-tetrahydronaphthalin
(+)-7-Amino-2-(N-butyl-N-propyl)amino-1,2,3,4-tetrahydronaphthalin
(-)-7-Amino-2-(N-phenethyl-N-propyl)amino-1,2,3,4-tetrahydronaphthalin
(+)-7-Amino-2-(N-phenethyl-N-propyl)amino-1,2,3,4-tetrahydronaphthalin
(-)-7-Amino-2-[N-(3-phenylpropyl)-N-propyl]amino-1,2,3,4-tetrahydronaphthalin
(+)-7-Amino-2-[N-(3-phenylpropyl)-N-propyl]amino-1,2,3,4-tetrahydronaphthalin
(-)-7-Amino-2-[N-(3-phenylallyl)N-propyl]amino-1,2,3,4-tetrahydronaphthalin
(+)-7-Amino-2-[N-(3-phenylallyl)N-propyl]amino-1,2,3,4-tetrahydronaphthalin
7-Dimethylamino-2-dipropylamino-1,2,3,4-tetrahydronaphthalin
2-Dipropylamino-7-methylamino-1,2,3,4-tetrahydronaphthalin
7-Dimethylamino-2-(N-phenethyl-N-propyl)amino-1,2,3,4-tetrahydronaphthalin
7-Methylamino-2-(N-phenethyl-N-propyl)amino-1,2,3,4-tetrahydronaphthalin
7-Amino-2-tert.-butoxycarbonylamido-1,2,3,4-tetrahydronaphthalin
7-Acetamido-2-tert.-butoxycarbonylamido-1,2,3,4-tetrahydronaphthalin
7-Acetamido-2-amino-1,2,3,4-tetrahydronaphthalin
7-Acetamido-2-(3-phenylallyl)amino-1,2,3,4-tetrahydronaphthalin
7-Acetamido-2-benzylamino-1,2,3,4-tetrahydronaphthalin
7-Acetamido-2-N,N-diethylamino-1,2,3,4-tetrahydronaphthalin
7-Acetamido-2-(N-allyl-N-methyl)amino-1,2,3,4-tetrahydronaphthalin
7-Acetamido-2-[N-methyl-N-(3-phenylallyl)]amino-1,2,3,4-tetrahydronaphthalin
7-Amino-2-ethylamino-1,2,3,4-tetrahydronaphthalin
2-Dipropylamino-7-methansulfonamido-1,2,3,4-tetrahydronaphthalin
2-(N-Phenethyl-N-propyl)amino-7-trifluoracetamido-1,2,3,4-tetrahydronaphthalin
7-Formamido-2-(N-phenethyl-N-propyl)amino-1,2,3,4-tetrahydronaphthalin
7-Carbamoylamido-2-dipropylamino-1,2,3,4-tetrahydronaphthalin
2-Dipropylamino-7-(N-methyl-formamido)-1,2,3,4-tetrahydronaphthalin
2-Dipropylamino-7-(N-methyl-acetamido)-1,2,3,4-tetrahydronaphthalin
2-Dipropylamino-7-ethylamino-1,2,3,4-tetrahydronaphthalin
7-Acetamido-2-methylamino-1,2,3,4-tetrahydronaphthalin
7-(N-Methyl-acetamido)-2-(N-phenethyl-N-propyl)amino-1,2,3,4-tetrahydronaphthalin

2-N,N-Dipropylamino-7-(N-propyl-acetamido)-1,2,3,4-tetrahydronaphthalin

Die neuen Verbindungen der allgemeinen Formel (I) wie auch die in Anspruch 1 ausgegrenzten Verbindungen eignen sich als Antipsychotika sowie als Sedativa.

Die neuen Verbindungen bzw. ihre physiologisch verträglichen Säureadditionssalze besitzen wertvolle pharmakologische Eigenschaften und weisen Wirkungen auf den Blutdruck und Herzfrequenz und auf die Prolaktinsekretion und insbesondere auf das Zentralnervensystem auf. Die erfindungsgemäßen Verbindungen zeigen eine präsynaptisch Dopamin agonistische Wirkung bei gleichzeitigem Fehlen einer postsynaptisch dopaminergen Wirkung. Aufgrund dieser Eigenschaften sind die Nebenwirkungen der bisher eingesetzten Antipsychotika (Neuroleptika) nicht zu erwarten. Dieses neuartige Wirkungsprofil macht die erfindungsgemäßen Verbindungen insbesondere zur Behandlung der Schizophrenie geeignet.

Als wertvoll haben sich dabei die Verbindungen bzw. Hydrochloride der allgemeinen Formel I herausgestellt, bei denen $R^1$ Wasserstoff, Methyl, Ethyl, Propyl, Butyl oder Allyl, $R^2$ einen Alkylrest - wie Propyl oder Butyl - oder einen Alkenylrest - wie Allyl - oder einen Alkinylrest - wie Propargyl - oder einen Aralkylrest - wie Phenethyl oder Thienylethyl -bedeutet und $R^3$ eine Acetyl- oder halogensubstituierte Acetylgruppe und $R^4$ Wasserstoff oder Niederalkyl bedeutet.

Besonders wertvoll erweisen sich beisipelsweise:

A: 7-Acetamido-2-{N-propyl-N-[2-(3-thienyl)ethyl]}-amino-1,2,3,4-tetrahydronaphthalin-Hydrochlorid
B: 7-Acetamido-2-(N-butyl-N-propyl)amino-1,2,3,4-tetrahydronaphthalin-Hydrochlorid
C: 7-Acetamido-2-(N-allyl-N-propyl)amino-1,2,3,4-tetrahydronaphthalin-Hydrochlorid
D: 7-Acetamido-2-(N-phenethyl-N-propyl)amino-1,2,3,4-tetrahydronaphthalin-Hydrochlorid
E: 7-Acetamido-2-(N-pentyl-N-propyl)amino-1,2,3,4-tetrahydronaphthalin
F: 7-Acetamido-2-(N-propargyl-N-propyl)amino-1,2,3,4-tetrahydronaphthalin

(In den nachfolgenden Tabellen werden anstelle der Verbindungsnamen die diesen zugeordneten Buchstaben -gegebenenfalls ergänzt durch die Angabe des optischen Isomeren (+) bzw. (-) - verwandt).

Zur Untersuchung der Beeinflussung präsynaptisch dopaminerger Rezeptoren die Wirkung auf die exploratorische Aktivität von Mäusen und die Hemmung der Dopaminsynthese bestimmt. Die Wirkung auf postsynaptische Dopaminrezeptoren wurde am Affen (MPTP-Modell) bzw. an der 6-OHDA- geschädigten Ratte bestimmt.

Hemmung der nächtlichen Motilität von Mäusen

Die Messung der Motilität einzelner weiblicher Mäuse des Stammes Chbl:NMR (SPF) (Gewicht 20 g) erfolgt in Käfigen (42 x 24 x 15 cm) mit horizontalen Lichtschranken. Registriert werden alle Lichtschranken-Unterbrechungen in Stunden-Intervallen mit Computer-on-line-Erfassung über 14 Stunden einer Nacht. Die Substanz wird unmittelbar von Meßbeginn sub cutan in 0,9 proz. NaCl-Lösung appliziert. Parallel zu den mit Substanz behandelten Tieren werden Placebo-behandelte Kontrolltiere mit untersucht. Bei der Auswertung wird aus den Ergebnissen von jeweils 5 Tieren pro Behandlungsgruppe der Mittelwert und die Standardabweichung bestimmt.

Die Ergebnisse sind in folgender Tabelle zusammengestellt:

Tabelle 1

| Hemmung der nächtlichen Motilität | | | |
|---|---|---|---|
| Substanz | untersuchter Dosisbereich (mg/kg s.c.) | erste wirksame Dosis (mg/kg s.c.) | Wirkzeit nach Applikation (h) |
| B | 0,1 - 10 | 1,0 | 0 - 8 |
| C | 0,1 - 10 | 10,0 | 0 - 3 |
| (+)-C | 0,1 - 10 | 10,0 | 0 - 3 |
| D | 0,1 - 10 | 10,0 | 0 - 7 |
| (+)-D | 0,1 - 10 | 10,0 | 0 - 7 |
| E | 0,1 - 10 | 10,0 | 0 - 4 |
| F | 0,1 - 10 | 10,0 | 0 - 5 |

Bestimmung der Dopaminsynthesehemmung

Die Methodik wird gemäß J.R. Walters und R.H. Roth [Naunyn - Schmiedeberg's Arch. Pharmacol. 296, (1976) 5] durchgeführt: Hierzu erhalten 5 Tiere jeweils 10 mg/kg s.c. der zu untersuchenden Substanz. Nach 5 Minuten erfolgt die Gabe von 750 mg/kg i.p. γ-Butyrolacton, um über die Blockade der präsynaptischen Impulsleitung den Einfluß postsynaptischer Rückkopplungsschleifen auf die Dopaminsyntheserate auszuschließen. Die Gabe von γ-Butyrolacton führt zu einer beträchtlichen Steigerung der DOPA- bzw. Dopaminsynthese. Zur Hemmung der Decarboxylierung von DOPA werden nach weiteren 5 Minuten 200 mg/kg i.p. 3-Hydroxybenzyl-hydrazin-Hydrochlorid appliziert. 40 Minuten nach Substanzgabe werden die Tiere getötet und das Corpus striatum präpariert. Die Messung des DOPA-Gehaltes erfolgt mit Hilfe von HPLC mit elektrochemischer Detektion (Standard: Dihydroxybenzylamin).

Bestimmt wird die durch die Testsubstanz bewirkte prozentuale Hemmung der durch γ-Butyrolacton stimulierten Dopaakkumulierung gegenüber den mit 0,9 proz. Kochsalzlösung behandelten Kontrolltieren.

Die Ergebnisse dieses Versuches sind in nachfolgender Tabelle zusammengestellt:

Tabelle 2

| Substanz | Dosis (mg/kg s.c.) | Hemmung der Dopaakkumulierung in % gegenüber den mit Kochsalz behandelten Kontrolltieren |
|---|---|---|
| A | 10 | 79 |
| B | 10 | 82 |
| (+)-B | 10 | 89 |
| (-)-B | 10 | 47 |
| C | 10 | 63 |
| (+)-C | 10 | 71 |
| D | 10 | 73 |
| (+)-D | 10 | 68 |
| (-)-D | 10 | 34 |
| E | 10 | 87 |
| F | 10 | 60 |

Bestimmung der postsynaptischen dopaminergen Wirkung mittels MPTP - Modell

Die pharmakologischen Eigenschaften des Neurotoxins 1-Methyl-4-phenyl-1,2,5,6-tetrahydro-pyridin (MPTP) [J.W. Langston, P. Bollard, J.W. Tetrud und I. Irwin, Science 219, (1983) 979] ermöglichen dessen Einsatz im Tiermodell für die Parkinsonsche Erkrankung.

Das durch MPTP beim Menschen und beim Affen ausgelöste, irreversible, neurologische Krankheitsbild ähnelt in seiner klinischen, pathologischen, biochemischen und pharmakologischen Ausprägung weitgehend der idiopathischen Parkinsonschen Erkrankung [S.D. Markey, J.N. Johannessen, C.C. Chivek, R.S. Burns und M.A. Herkenham, Nature 311, (1984) 464]. Ursache dieser überzeugenden Übereinstimmung ist die Tatsache, daß MPTP selektiv kleine Gruppen dopaminerger Nervenzellen in der Substantia nigra des Gehirns zerstört, welche auch bei der natürlich auftretenden Parkinsonschen Erkrankung durch degenerative Prozesse zerstört werden.

Es besteht die Möglichkeit, daß die Ursache der idiopathischen Parkinsonschen Erkrankung im Organismus entstehendes MPTP oder eine ähnliche chemische Verbindung ist [S.H. Snyder, Nature 311 - (1984) 514]. Die klinische Ausprägung des MPTP-Parkinsonbildes ist - möglicherweise bedingt durch den spezifischen Metabolismus des MPTP -bisher außer beim Menschen nur beim Affen nachweisbar.

Das beim Rhesusaffen verwirklichte MPTP-Modell ist daher geeignet, die Wirkung von postsynaptisch dopaminagonistisch wirkenden Substanzen zu prüfen.

Dazu erhalten Rhesusaffen MPTP in Gesamtdosen bis etwa 6 mg/kg Körpergewicht bis folgende Symptome auftreten: die Tiere werden akinetisch und sind nicht in der Lage, Wasser und Futter aufzunehmen. Sie zeigen eine typische, gebeugte Haltung; gelegentlich treten kataleptische Zustände auf. Die Extremitäten weisen einen Rigor auf, welcher bei passiver Bewegung von klonischen Krämpfen durchbrochen wird. Dopamin-Agonisten, wie B-HT 920, Levadopa oder Apomorphin führen zu einer vorübergehenden Aufhebung dieses Zustandsbildes für die Zeit von 4 bis 5 Stunden bei einer einmaligen Dosis von 100 µg/kg intramuskulär. Danach tritt erneut die oben beschriebene Symptomatik auf.

Nach der intramuskulären Gabe der erfindungsgemäßen Verbindungen (0,1 - 3,0 mg/kg) kann keine Verbesserung oder gar Aufhebung des Zustandsbildes der mit MPTP behandelten Affen beobachtet werden. Bei einigen der erfindungsgemäßen Verbindungen beobachtet man eine Antagonisierung der durch B-HT 920 hervorgerufenen Aufhebung der Parkinson ähnlichen Symptomatik. Die Tiere bleiben bei der Gabe von 1,00 - 3,0 mg/kg der erfindungsgemäßen Verbindungen zusammen mit 0,1 mg/kg B-HT 920 bradykinetisch und sind weiterhin nicht in der Lage Wasser und Futter aufzunehmen. Daraus ist auf eine postsynaptisch dopaminantagonistische Wirkung dieser Substanzen zu schließen.

Die Ergebnisse sind in der folgenden Tabelle zusammengefaßt:

Tabelle 3

| Substanz | Dosis | Wirkung |
|---|---|---|
| A | bis 2 mg/kg | keine Aufhebung der MPTP-Symptomatik |
| B | 3 mg/kg | Antagonisierung von B-HT 920* |
| (+)-B | 0,6 mg/kg | Antagonisierung von B-HT 920* |
| (-)-B | 2 mg/kg | Antagonisierung von B-HT 920* |
| C | 3 mg/kg | Antagonisierung von B-HT 920* |
| (+)-C | 1 mg/kg | Antagonisierung von B-HT 920* |
| D | 1 mg/kg | Antagonisierung von B-HT 920* |
| (+)-D | 0,5 mg/kg | Antagonisierung von B-HT 920* |
| E | 3 mg/kg | Antagonisierung von B-HT 920* |
| F | 1 mg/kg | Antagonisierung von B-HT 920* |

* 0,1 mg B-HT 920 pro kg Körpergewicht

Bestimmung der postsynaptischen dopaminergen Wirkung mittels 6-OH-Dopamin- (OHDA)-geschädigter Ratten

Bei Ratten wird durch Injektion von 6-OHDA in das mediale Vorderhirnbündel die nigrostriatale Bahn unterbrochen. Erfolgreich geschädigte Ratten werden durch Testgaben von Apomorphin selektiert. Das Rotationsverhalten wird mit einem modifizierten Rotameter nach Ungerstedt und Arbuthnott [Brain Res. 24, (1970) 485] bestimmt, wobei die durch die Testsubstanz bewirkte prozentuale Reduzierung der durch eine Verabreichung von 0,1 mg/kg B-HT 920 hervorgerufenen kontralateralen Drehungen gemessen wird.

Die Ergebnisse sind in folgender Tabelle zusammengestellt:

Tabelle 4

| Substanz | Dosis mg/kg | Reduzierung der Drehungen im Zeitraum | |
|---|---|---|---|
| | | 15′-30′ | und 60′-75′ nach Substanzgabe |
| B | 3 | 22 % | 95 % |
| (+)-B | 3 | 93 % | 99 % |
| C | 3 | 86 % | 93 % |
| (+)-C | 1 | 71 % | 49 % |
| D | 3 | 100 % | 82 % |
| (+)-D | 1 | 43 % | 76 % |
| (-)-D | 3 | 47 % | 73 % |
| F | 3 | 43 % | 36 % |

I

Das als Ausgangsmaterial verwandte 2,7-Diamino-1,2,3,4-tetrahydronaphthalin kann nach bekannten Methoden hergestellt [S. Chiavarelli und G. Settimj, Rend. ist. super. sanità 22 (1959) 508; C.A. 54 (1960) 2281 g] und mit den üblichen Methoden - beispielsweise über die entsprechenden Weinsäuresalze - in die beiden Enantiomeren getrennt werden.

II

Der für die weitere Synthese notwendige Schutz der Aminofunktion ($R_S$) läßt sich unter Anwendung der aus dem Stand der Technik bekannten Methoden erzielen [M. Bodanszky, Principles of Peptide-Synthesis, Springer-Verlag, Berlin 1984, S. 84; T.W. Greene, Protective Groups in Organic Synthesis, John Wiley and Sons, New York 1981, S. 218 ff; Houben-Weyl, Methoden der organischen Chemie, Band E 4, Georg Thieme Verlag, Stuttgart 1983, S. 144], wobei der Schutz der Aminofunktion mit einer Alkoxycarbonyl- oder einer Aryloxycarbonyl-Schutzgruppe bevorzugt wird und wobei die tert.-Butoxycarbonyl- (BOC-) Schutzgruppe besonders bevorzugt wird.

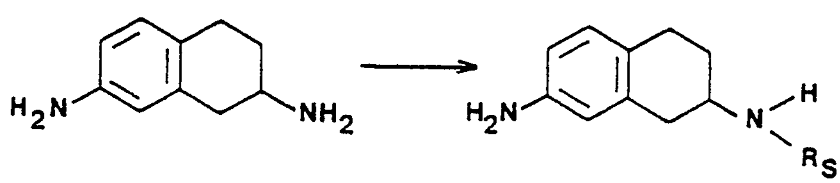

(IV)

So lassen sich beispielsweise unter Anwendung an sich bekannter Methoden - mit Pyrokohlensäure-di-tert.-butylester in Gegenwart einer basisch reagierenden Verbindung - vorzugsweise einer organischen Stickstoffbase wie z.B. Triethylamin in inerten Lösungsmitteln - die entsprechenden enantiomerenreinen 7-Amino-2-tert.-butoxycarbonylamido-1,2,3,4-tetrahydronaphthaline darstellen. Als inerte Lösungsmittel dienen im allgemeinen organische Lösungsmittel, die sich unter den angewandten Reaktionsbedingungen nicht verändern. Hierzu gehören bevorzugt Ether wie zum Beispiel Tetrahydrofuran oder Glykoldimethylether (Glyme).

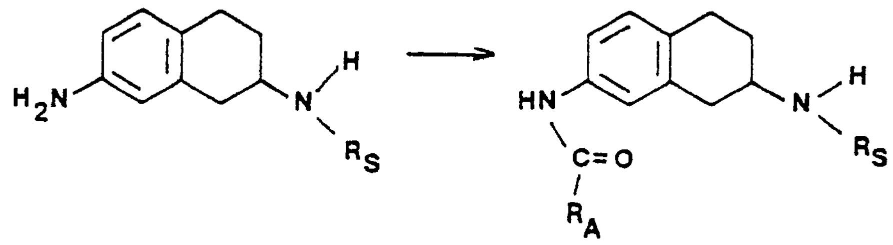

(IV) (V)

$R_A$ = H, Alkyl

III

Das jeweilige geschützte R- bzw. S-Diamino-1,2,3,4-tetrahydronaphthalin-Derivat vorzugsweise das R- bzw. S-7-Amino-2-tert.-butoxycarbonylamido-1,2,3,4-tetrahydronaphthalin der allgemeinen Formel IV läßt sich mit den aus dem Stand der Technik bekannten sowie insbesondere zur Herstellung von Acylaniliden geeigneten Verfahren in die entsprechend geschützten 7-Acylamido-2-amino-1,2,3,4-tetrahydronaphthalin-Derivate bzw. 7-Acylamido-2-tert.-butoxycarbonylamido-1,2,3,4-tetrahydronaphthaline überführen, welche unter die allgemein Formel V fallen [C. Ferri, Reaktionen der organischen Synthese, Georg Thieme Verlag, Stuttgart 1978, S. 222 ff].

Hierbei wird die Umsetzung des jeweiligen geschützten 2,7-Diamino-1,2,3,4-tetrahydronaphthalin-Derivates bzw. des bevorzugten 7-Amino-2-tert.-butoxycarbonylamido-1,2,3,4-tetrahydronaphthalins mit einem reaktiven Carbonsäurederivat in einem inerten Lösungsmittel sowie in Gegenwart einer Base bevorzugt. Unter reaktiven Carbonsäurederivaten sind im allgemeinen Carbonsäurehalogenide sowie Carbonsäureanhydride zu verstehen, die gegebenenfalls mit Halogenatomen substituiert sein können. Derartige Carbonsäurederivate sind bekannt oder können nach bekannten Methoden hergestellt werden [Houben-Weyl, Methoden der organischen Chemie, Band VIII und Band E 5, Georg Thieme Verlag, Stuttgart 1952 bzw. 1985].

Bevorzugt sind hierbei aliphatische Carbonsäurebromide, Carbonsäurechloride oder aliphatische Carbonsäureanhydride. Besonders bevorzugt sind Essigsäurebromid, Essigsäurechlorid, Essigsäureanhydrid und Trifluoressigsäureanhydrid.

Als Basen können alle üblichen basischen Verbindungen, die für eine basische Reaktionsführung geeignet sind, eingesetzt werden. Hierzu gehören beispielsweise Alkali- oder Erdalkalihydroxide, -carbonate oder -alkoholate und vorzugsweise tertiäre Amine, wobei besonders bevorzugt Triethylamin Verwendung findet.

Als inerte Lösungsmittel werden im allgemeinen organische Solvenzien eingesetzt, die sich unter den angewandten Reaktionsbedingungen nicht verändern wie zum Beispiel Kohlenwasserstoffe - wie Benzol, Toluol, Xylol oder Erdölfraktionen - oder Halogenkohlenwasserstoffe - wie z.B. Methylenchlorid, Chloroform oder Tetrachlorkohlenstoff oder bevorzugt Ether - wie Diethylether, Glykoldimethylether (Glyme), Diglykoldimethylether (Diglyme) und besonders bevorzugt Tetrahydrofuran.

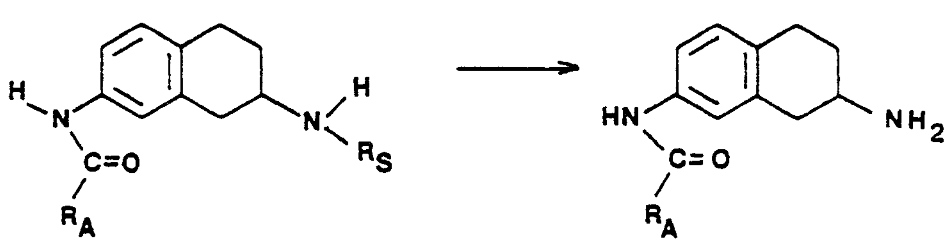

(V) (VI)

IV

Die nachfolgende Abspaltung der Schutzgruppe $R_S$ von der Aminofunktion in 2-Stellung im Tetrahydronaphthalin der allgemeinen Formel V kann - je nach Art der Schutzgruppe und in Abhängigkeit von der chemischen Stabilität der Acylanilid-Teilstruktur - unter Anwendung der aus dem Stand der Technik bekannten Verfahren erfolgen [T.W. Greene, Protective Groups in Organic Synthesis, John Wiley and Sons, New York 1981, S 218 ff; U. Petersen in Houben-Weyl, Methoden der organischen Chemie, Band E4, Georg Thieme Verlag, Stuttgart 1983, S. 144], woraus das acylierte 1,2,3,4-Tetrahydronaphthalin-Derivat der allgemeinen Formel VI resultiert.

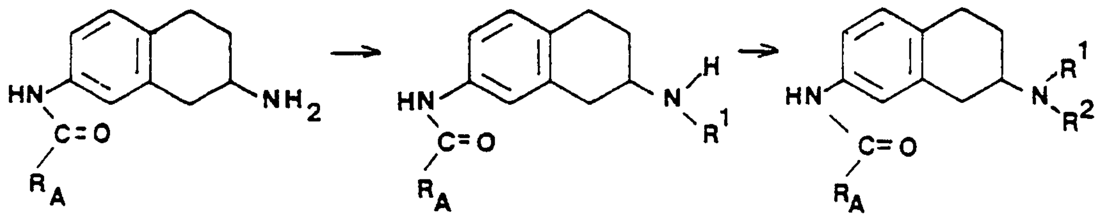

(VI) (VII) (VIII)

V

Für die Überführung der primären Aminofunktion im 7-Acylamino-2-amino-1,2,3,4-tetrahydronaphthalin-Derivat der allgemeinen Formel VI in ein sekundäres Amin -woraus ein Derivat der allgemeinen Formel VII resultiert - oder in ein tertiäres Amin - woraus ein Tetrahydronaphthalin-Derivat der allgemeinen Formel VIII resultiert - sind aus dem Stand der Technik ebenfalls zahlreiche Verfahren bekannt [G. Spielberger in Houben-Weyl, Methoden der organischen Chemie, Band XI/1, Georg Thieme Verlag, Stuttgart 1957, S. 24; J. March, Advanced Organic Chemistry, 3rd Ed., John Wiley and Sons, New York 1985, S. 1153].

Bevorzugt wird zum einen die Alkylierung mit gegebenenfalls derivatisierten Alkyl- bzw. Aralkyhalogeniden beziehungsweise -sulfaten, -tosylaten oder -trifluormethansulfonaten durchgeführt - wie z.B. Methyl-, Ethyl-, Propyl-, Isopropyl-, Butyl-, tert.-Butyl, Allyl-, Propargyl-, Benzyl-, Phenethyl-, o-, m-, p-Methylbenzyl-, o-, m-, p-Methoxybenzyl-omega-Bromstyryl-halogeniden,
1-Halogen-2-(2-thienyl)-ethanen,
1-Halogen-2-(3-thienyl)-ethanen,
1-Halogen-3-phenylpropanen
1-Halogen-3-phenyl-prop-2-enen,
1-Halogen-4-phenylbutanen,
Halogenmethylcyclopropanen,
1-Halogen-3-(p-chlorphenyl)-propanen,
(2-Halogenethyl)-methylethern,
(2-Halogenethyl)-phenylethern (omega-Halogenphenetolen),
1-Halogen-3-methoxypropanen,
(2-Halogenethyl)-methylsulfiden,
(2-Halogenethyl)-phenylsulfiden,
(3-Halogenpropyl)-methylsulfiden,
$\beta$-Halogenpropionsäuremethylestern,
$\beta$-Halogenpropionsäurephenylestern,
$\beta$-Halogenpropionsäureestern,
$\beta$-Halogenpropionsäureamiden,
4-Halogenbutan-2-onen,
2-(2-Halogenethyl)-furanen,
3-(2-Halogenethyl)-furanen,
2-(2-Halogenethyl)-pyridinen,
3-(2-Halogenethyl)-pyridinen,
p-(2-Halogenethyl)-methoxybenzolen,
p-(2-Halogenethyl)-toluolen,

p-(2-Halogenethyl)-methoxybenzolen,
(2-Halogenethyl)-3,4-dimethoxybenzolen,
(2-Halogenethyl)-3-chlorbenzolen,
(2-Halogenethyl)-2,4-dichlorbenzolen,
(2-Halogenethyl)-3,5-dichlorbenzolen,
1-(2-Halogenethyl)-naphthalinen,
2-(2-Halogenethyl)-naphthalinen,
(2-Halogen-1-methylethyl)-benzolen,
(2-Halogenethyl)-cyclohexanen,
2-(3-Halogenprop-2-en-1-yl)-pyridinen,
wobei Halogen bzw. -halogenid Chlor, Brom, Jod und in zweiter Linie Fluor bedeutet. Anstelle von Halogen bzw. -halogenid kann aber auch beispielsweise eine Sulfathalbester-, eine Methansulfonsäureester Trifluormethylsulfonester- oder eine Tosylatgruppe stehen.

Derartige Halogenide, Sulfate, Trifluormethylsulfonate oder Tosylate sind bekannt oder können nach bekannten Methoden hergestellt werden, wobei beim Einbau von polyfunktionellen Substituenten, die z.B. freie phenolische Hydroxyl- oder (Carbon)säurefunktionen aufweisen, zunächst deren entsprechend geschützte Derivate - wie z.B. Ether bzw. Ester oder Amide eingeführt werden, die in einem weiteren Schritt auf konventionelle Weise - z.B. durch Ether- oder Esterspaltung - bzw. durch Verseifung in die gewünschten Derivate überführt werden. Zu solchen Derivaten gehören beispielsweise 1-Brom-2-(4-methoxybenzol)ethan, Bromessigsäureethylester, und 3-Brompropionsäurediethylamid. Als Lösungsmittel eignen sich alle inerten organischen Lösungsmittel, die sich unter den Reaktionsbedingungen nicht verändern oder selbst nicht nachteilig als reaktionsfähige Komponenten in den Reaktionsablauf eingreifen können. Hierzu gehören bevorzugt Alkohole - wie Methanol, Ethanol, Propanol oder Isopropanol - oder Ester - wie Essigsäuremethylester und Essigsäureethylester, Halogenkohlenwasserstoffe sofern sie selbst nicht als Alkylierungsagenz wirken - bevorzugt Fluorkohlenwasserstoffe und besonders bevorzugt Ether wie Diethylether, Di-n-butylether, tert.-Butylmethylether, Glykoldimethylether (Glyme), Diethylenglykoldimethylether (Diglyme), Tetrahydrofuran, Dioxan und Säureamide wie zum Beispiel Hexamethylphosphorsäuretriamid oder Dimethylformamid.

Außerdem ist es möglich, Gemische der genannten Lösungsmittel zu verwenden.

Die Reaktion wird vorzugsweise in Gegenwart von säurebindenden Agenzien - wie z.B. Alkali- oder Erdalkalicarbonaten oder -hydrogencarbonaten durchgeführt.

VI

Zum anderen kann die primäre Aminofunktion des Diamins der allgemeinen Formel VII auf dem Wege der reduktiven Aminierung eines geeigneten Aldehyds oder Ketons bzw. von deren geeigneten Derivaten - in Abhängigkeit vom Molverhältnis der eingesetzten Carbonylverbindung zum 7-Acylamino-2-amino-1,2,3,4-tetrahydronaphthalin-Derivat der allgemeinen Formel VI - in das entsprechende sekundäre oder tertiäre Amin überführt werden.

Die als Edukte eingesetzten Aldehyde bzw. Ketone sind bekannt oder können nach bekannten Methoden hergestellt werden [Aldehyde: Houben-Weyl, Methoden der organischen Chemie, Band VII/1 und E5, Georg Thieme Verlag, Stuttgart 1954 bzw. 1983; Ketone: Houben Weyl, Methoden der organischen Chemie, Band VII/2a und 2b, Georg Thieme Verlag, Stuttgart 1973 bzw. 1976].

Geeignete Aldehyde bzw. Ketone werden z.B. durch Formaldehyd, Acetaldehyd, Propionaldehyd, Aceton, Butyraldehyd, Acrolein, Propargylaldehyd, Benzaldehyd, 1-Naphthaldehyd, 2-Naphthaldehyd, Phenylacetaldehyd, 2-Phenylpropionaldehyd, 3-Phenylpropionaldehyd, 3-Phenylacrolein (Zimtaldehyd), 4-Phenylbutyraldehyd, Cyclopropylformaldehyd, Cyclopentylaldehyd, Cyclohexylaldehyd, Methoxyacetaldehyd, Phenoxyacetaldehyd, Benzyloxyacetaldehyd, 3-Methoxypropionaldehyd, Methylmercaptoacetaldehyd, Phenylmercaptoacetaldehyd, 3-Methylmercaptopropionaldehyd, (4-Chlorphenyl)-acetaldehyd, (3,4-Dichlorphenyl)-acetaldehyd, 4-Methoxyacetaldehyd, (3,4-Dimethoxyphenyl)-acetaldehyd, (3,5-Dimethoxyphenyl)-acetaldehyd, (2-Furyl)-acetaldehyd, (3-Furyl)-acetaldehyd, (2-Pyridyl)-acetaldehyd, (3-Pyridyl)-acetaldehyd, (4-Pyridyl)-acetaldehyd, (1-Naphthyl)-acetaldehyd, (2-Naphthyl)-acetaldehyd und 3-(2-Pyridyl)-acrolein verkörpert.

Die Herstellung erfolgt in inerten organischen Lösungsmitteln, gegebenenfalls in Anwesenheit eines Katalysators und erforderlichenfalls in Anwesenheit eines wasserbindenden Mittels.

Als inerte Solvenzien eignen sich hierbei inerte organische Lösungsmittel, die sich unter den gegebenen Reaktionsbedingungen nicht verändern und selbst nicht nachteilig in den Reaktionsablauf eingreifen.

Hierzu gehören vorzugsweise Alkohole - wie zum Beispiel Methanol, Ethanol, Propanol oder Isopropanol - oder Ether wie zum Beispiel Diethylether, tert.-Butylmethylether, Di-n-butylether, Glykoldimethylether (Glyme), Diethylenglykoldimethylether (Diglyme), Tetrahydrofuran - oder Halogenkohlenwasserstoffe - wie zum Beispiel Dichlormethan, Trichlormethan, Tetrachlormethan - oder Carbonsäureester - wie zum Beispiel Essigsäuremethylester und Essigsäureethylester - oder Kohlenwasserstoffe - wie zum Beispiel Erdölfraktionen, Benzol, Toluol, Xylol - und besonders bevorzugt Amide -wie zum Beispiel Dimethylformamid, Acetamid, Hexamethylphosphorsäuretriamid - oder Carbonsäuren -wie zum Beispiel Ameisensäure oder Essigsäure - bzw. auch Mischungen der genannten Lösungsmittel.

Als Katalysatoren werden gegebenenfalls Protonensäuren verwendet. Hierunter fallen bevorzugt Mineralsäuren wie zum Beispiel Salzsäure oder Schwefelsäure oder organische Carbonsäuren mit 1 bis 6 C-Atomen, die gegebenenfalls durch Fluor, Chlor und/oder Brom substituiert sein können. Beispiele derartiger Säuren sind Ameisensäure, Essigsäure, Trifluoressigsäure, Trichloressigsäure oder Propionsäure. Unter die Gruppe der bevorzugten Säuren fallen ebenso Sulfonsäuren mit $C_1$-$C_4$-Alkylresten oder Arylresten, die gegebenenfalls durch Halogenatome substituiert sein können - wie zum Beispiel Methansulfonsäure, Trifluormethansulfonsäure, Ethansulfonsäure, Benzolsulfonsäure, Toluolsulfonsäure.

Das bei der Reaktion entstehende Wasser kann erforderlichenfalls durch Zugabe von wasserbindenden Mitteln - wie zum Beispiel Phosphorpentoxid - oder bevorzugterweise durch Molekularsieb oder aber im Gemisch mit dem eingesetzten Lösungsmittel während oder nach dem Ende der Reaktion beispielsweise auf dem Wege der Destillation dem Reaktionsgemisch entzogen werden.

Die Reaktion wird im allgemeinen in einem Temperaturintervall von +20 °C bis zum Siedepunkt der jeweiligen Reaktionsmischung und bevorzugt in einem Temperaturbereich von +20 °C bis 100 °C durchgeführt.

Beim azeotropen Abdestillieren des bei der Reaktion gebildeten Wassers mit dem jeweils verwendeten Lösungsmittel bzw. Lösungsmittelgemisch wird die Siedetemperatur des jeweiligen Azeotrops bevorzugt.

Die Reaktion wird im allgemeinen bei Normaldruck durchgeführt, kann aber auch bei erhöhtem oder erniedrigtem Druck durchgeführt werden.

Zur Herstellung des sekundären Amins werden das 7-Acetamido-2-amino-1,2,3,4-tetrahydronaphthalin und die jeweilige Carbonylverbindung äquimolar eingesetzt. Zur Herstellung des tertiären Amins wird die jeweilige Carbonylverbindung im Überschuß eingesetzt. Des weiteren hat es sich bei der Darstellung von tertiären Aminen, welche zum Beispiel unter die allgemeine Formel VIII fallen, im zweistufigen Alkylierungen bei der zweiten reduktiven Alkylierung als besonders vorteilhaft erwiesen, Dimethylformamid als Reaktionsmedium zu verwenden.

Zur Erzielung höherer Ausbeuten erweist es sich bei der Darstellung der Tetrahydronaphthalinen der allgemeinen Formel VII gegebenenfalls als vorteilhaft, das Amin der allgemeinen Formel VI zunächst der reduktiven Aminierung mit Benzaldehyd zu unterwerfen.

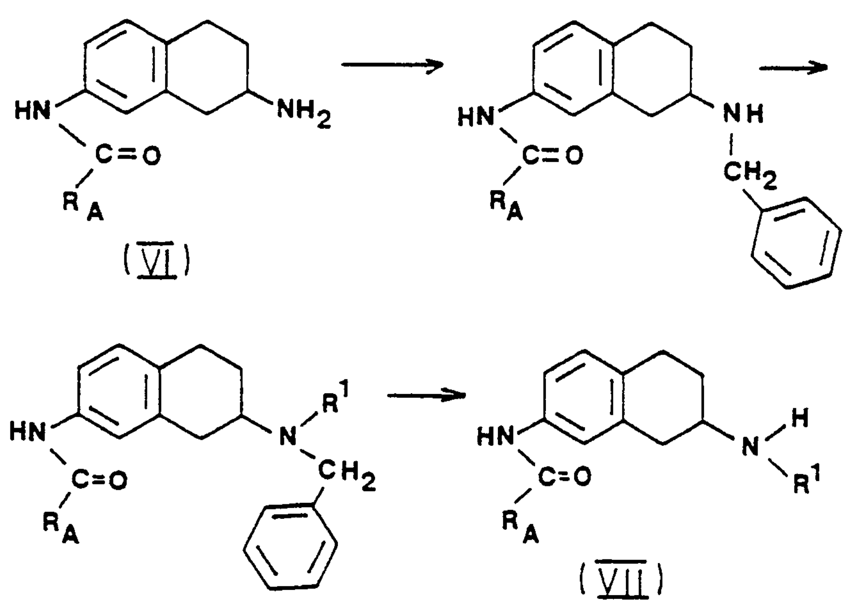

(VI)

(VII)

Die Benzyl-Schutzgruppe wird nach der Einführung des gewünschten Substituenten abgespalten. Die

reduktive Aminierung mit Benzaldehyd sowie die Abspaltung der Benzylschutzgruppe aus dem nach der weiteren Alkylierung resultierenden tertiären Amins ist aus dem Stand der Technik bekannt [T.W. Greene, Protective Groups in Organic Synthesis, John Wiley and Sons, New York 1981, S. 272 ff und zit. Lit.].

Die bei der Herstellung von sekundären bzw. tertiären Aminen als Zwischenprodukte anfallenden Schiff'schen Basen bzw. Enamine oder Immoniumsalze können entweder durch Wasserstoff in Wasser oder in inerten organischen Lösungsmitteln wie Alkoholen, Ethern oder Halogenkohlenwasserstoffen, oder deren Gemischen mit Wasserstoff in Gegenwart geeigneter Katalysatoren oder aber mit komplexen Hydriden - gegebenenfalls in Gegenwart eines Katalysators - hydriert bzw. reduziert werden. Dabei kann die Reduktion in dem selben Reaktionsmedium oder aber auch in einem anderen Lösungsmittel bzw. in einer anderen Lösungsmittelmischung erfolgen. Im letzteren Fall wird das zuerst eingesetzte Lösungsmittel bzw. Lösungsmittelgemisch bevorzugt durch Destillation entfernt.

Als Katalysatoren finden bei der katalytischen Reduktion mit Wasserstoff beispielsweise Raney-Nickel, Palladium, Palladium auf Tierkohle, Platin und bevorzugt Palladium auf Kohle (10 % Pd-Gehalt) Verwendung.

Bei der Reduktion mit Hydriden werden bevorzugt komplexe Hydride des Bors oder des Aluminiums eingesetzt. Besonders bevorzugt wird Lithiumaluminiumhydrid und Natriumborhydrid verwandt.

Als Lösungsmittel eignen sich alle inerten organischen Lösungsmittel, die unter den gewählten Reaktionsbedingungen unverändert oder zumindest weitgehend unverändert bleiben und die nicht nachteilig in den Reaktionsablauf eingreifen. Dazu gehören Amide -wie zum Beispiel Dimethylformamid oder Hexamethylphosphorsäuretriamid - oder Ester - wie Essigsäuremethylester oder Essigsäureethylester- oder Ether - wie beispielsweise Diethylether, tert.-Butylmethylether, Di-n-butylether, Glykoldimethylether (Glyme), Diglykoldimethylether (Diglyme), Tetrahydrofuran und Dioxan - und besonders bevorzugt Alkohole - beispielsweise Methanol, Ethanol, Propanol oder Isopropanol.

Als Katalysatoren werden gegebenfalls Protonensäuren verwendet. Hierunter fallen bevorzugt Mineralsäuren wie zum Beispiel Salzsäure oder Schwefelsäure oder organische Säuren mit 1 bis 6 C-Atomen, die gegebenenfalls durch Fluor, Chlor und/oder Brom substituiert sein können. Beispiele derartiger Säuren sind Ameisensäure, Essigsäure, Trifluoressigsäure, Trichloressigsäure oder Propionsäure. Zu den bevorzugten Säuren gehören ebenso Sulfonsäuren mit $C_1$-$C_4$-Alkylresten oder Arylresten, die gegebenenfalls durch Halogenatome substituiert sein können, wie zum Beispiel Methansulfonsäure, Trifluormethansulfonsäure, Ethansulfonsäure, Benzolsulfonsäure, Toluolsulfonsäure.

Bei der Reduktion mit Wasserstoff wird die Reaktion unter den für die katalytische Hydrierung in Abhängigkeit von den jeweils eingesetzten Katalysatoren, Lösungsmitteln und Reaktanden bekannten Reaktionsbedingungen durchgeführt [K. Harada in Patai, "The Chemistry of the Carbon-Nitrogen Double-Bond", Interscience Publishers, London 1970, S. 276 u. zit. Lit.; P.N. Rylander, Catalytic Hydrogenation over Platinum Metals, Academic Press, New York 1967, S. 123; F. Möller und R. Schröter in Houben-Weyl, Methoden der organischen Chemie, Band XI/1, Georg Thieme Verlag, Stuttgart 1957, S. 602; W.S. Emerson, Org. Reactions 4 (1949) 174; E.M. Hancock und A.C. Cope, Org. Synth., Coll. Vol. III (1955) 501; J.C. Robinson und H.R. Snyder, Org. Synth., Coll, Vol. III (1955) 717; D.M. Malcolm und C.R. Noller, Org. Synth., Coll. Vol. IV (1963) 603].

Bevorzugt wird die Hydrierung mit Palladium auf Kohle (10 % Pd-Gehalt) in Methanol in einem Temperaturbereich von 20 bis 50 °C - besonders bevorzugt in einem Temperaturintervall von 25 °C bis 35 °C - unter einem Wasserstoffdruck von vorzugsweise 0,1 bis 0,8 MPa -besonders bevorzugt unter einem Wasserstoffdruck von 0,4 bis 0,6 MPa - durchgeführt.

Bei der Durchführung des erfindungsgemäßen Verfahrens unter Verwendung von komplexen Hydriden hat es sich als vorteilhaft erwiesen, das Amin mit dem entsprechenden Aldehyd bzw. Keton in einer Eintopfreaktion unter Verwendung eines inerten Lösungsmittels vorzugsweise eines Carbonsäureamids und besonders bevorzugt unter Verwendung von Dimethylformamid in einem Temperaturbereich zwischen 50 °C und 100 °C umzusetzen, das Lösungsmittel nach beendeter Reaktion unter vermindertem Druck abzudestillieren, den verbleibenden Rückstand in einem organischen Lösungsmittel -vorzugsweise einem Alkohol - zu lösen bzw. zu dispergieren und mit einer mindestens äquimolaren Menge eines Reduktionsmittels - vorzugsweise eines komplexen Hydrides und besonders bevorzugt Natriumborhydrid -umzusetzen, nach erfolgter Reduktion hydrolytisch aufzuarbeiten, das Reaktionsprodukt auf extraktivem Wege aus dem Reaktionsgemisch zu isolieren und ggf. chromatographisch zu reinigen oder auf anderem Wege einen Reinigungsschritt zu unterwerfen und zu isolieren.

VII

Die Herstellung der erfindungsgemäßen Verbindungen im Rahmen einer reduktiven Aminierung ist in einer weiteren Synthesevariante auf dem Wege einer Leukart-Wallach-Reaktion durchführbar, welche sich besonders zur Darstellung tertiärer Amide eignet. Die Reaktionsbedingungen für derartige reduktive Aminierungen sind bekannt [Autorenkolektiv, Organikum, VEB Deutscher Verlag der Wissenschaften, Berlin 1986 (16. Auflage) S. 491; C. Ferri, Reaktionen der organischen Synthese, Georg Thieme Verlag, Stuttgart 1978, S. 133 und zit. Lit.; F. Möller und R. Schröter in Houben-Weyl, Methoden der organischen Chemie, Band XI/1, Georg Thieme Verlag, Stuttgart 1957, S. 648].

Des weiteren können die erfindungsgemäßen Verbindungen durch jede andere Umsetzung, die von primären Aminen als Aminkomponente ausgeht - beispielsweise auf dem Wege einer Mannich-Reaktion dargestellt werden [C. Ferri, Reaktionen der organischen Synthese, Georg Thieme Verlag, Stuttgart 1978, S. 302 ff und 496 ff].

Die erfindungsgemäßen tertiären Amine der allgemeinen Formel VIII können ebenso durch Kombination der genannten Herstellungsmethoden erhalten werden.

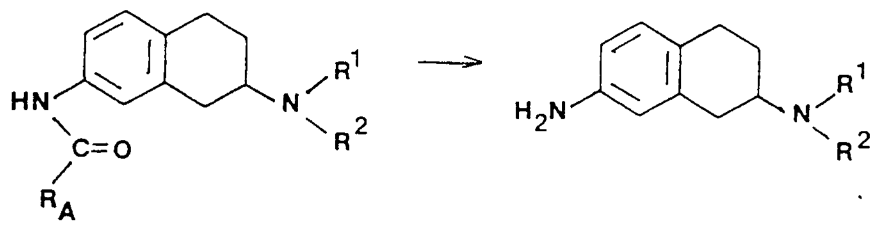

(VII): $R^1 \neq H$, $R^2 = H$
(VIII): $R^1 \neq H$, $R^2 \neq H$

(IX): $R^1 \neq H$, $R^2 = H$
(X): $R^1 \neq H$, $R^2 \neq H$

VIII

Die Acyl-Gruppe kann zur weiteren Derivatisierung der anilinischen Aminofunktion abgespalten werden, woraus die Diamine der allgemeinen Formeln IX und X resultieren.

Die Verseifung derartiger Arylamide ist bekannt und kann durch Umsetzung eines Carbonsäureamids der allgemeinen Formel VII oder VIII beispielsweise durch Umsetzung der Säureamide mit wässerigen Lösungen von Alkalihydroxiden oder Säuren erzielt werden [F. Möller in Houben-Weyl, Methoden der organischen Chemie, Band XI/1, Georg Thieme Verlag Stuttgart 1957, S. 927 ff und 934 ff; R.M. Herbst und D. Shemin, Org. Synth., Coll. Vol. II (1943) 491; P.E. Fanta und D.S. Tarbell, Org. Synth., Coll. Vol. III (1955) 661]. Bevorzugt wird die Umsetzung mit verdünnter Mineralsäuren unter Rückflußbedingungen, wobei die Verseifung mit 2N Salzsäure besonders bevorzugt wird, die in einer Eintopfreaktion sofort die entsprechenden Hydrochloride liefert, welche nach dem Abdestillieren des Reaktionsmediums und der flüchtigen Bestandteile der Reaktionsmischung - vorzugsweise unter vermindertem Druck - und dem Aufschlämmen in einem organischen Lösungsmittel - vorzugsweise einem Alkohol, wobei Ethanol besonders bevorzugt wird - sowie nach dem Filtrieren und Trocknen isoliert werden können.

IX

Sofern eine Alkylierung des aus der Abspaltung resultierenden freien anilinischen Stickstoffes gewünscht wird, kann diese - in Abhängigkeit davon, ob ein sekundäres oder tertiäres Amin angestrebt wird -mit den unter V und/oder VI und/oder VII angegebenen Methoden erfolgen, woraus di-, tri- oder tetrasubstituierte 2,7-Diamino-1,2,3,4-tetrahydronaphthalin-Derivate der allgemeinen Formel XII, XIII, XIV oder XV resultieren.

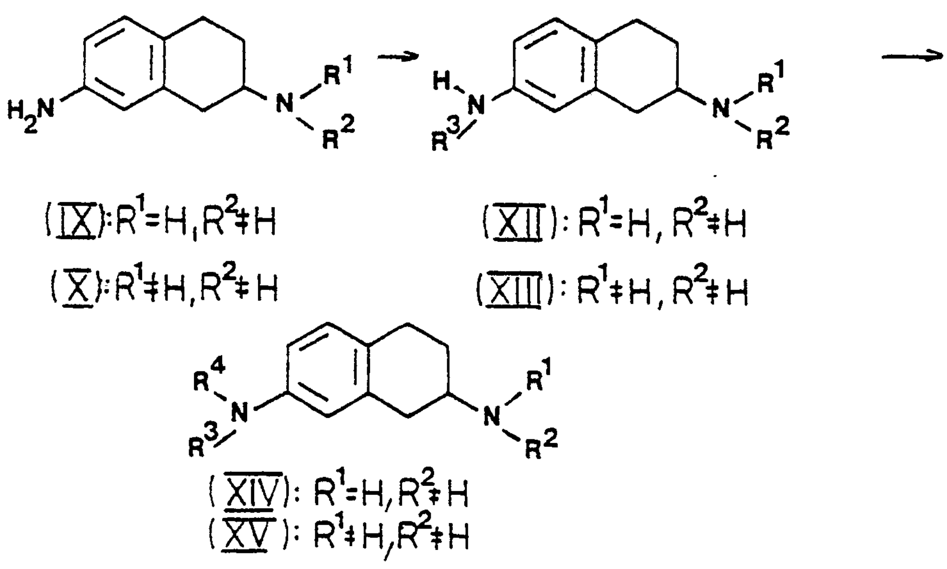

(IX): $R^1 = H, R^2 \neq H$

(X): $R^1 \neq H, R^2 \neq H$

(XII): $R^1 = H, R^2 \neq H$

(XIII): $R^1 \neq H, R^2 \neq H$

(XIV): $R^1 = H, R^2 \neq H$

(XV): $R^1 \neq H, R^2 \neq H$

X

Eine Formylierung oder eine erneute Acylierung der 2,7-Diamino-1,2,3,4-tetrahydronaphthalin-Derivate der allgemeinen Formel X kann z.B. mit den unter III angegebenen Methoden oder mit der ebenfalls aus dem Stand der Technik wohlbekannten Aminolyse der entsprechend gewünschten Carbonsäureester [J. March, Advanced Organic Chemistry, 3rd Ed., John Wiley and Sons, New York 1985, S. 375 und zit. Lit.] erfolgen.

XI

Des weiteren werden durch Umsetzung der 2,7-Diamino-1,2,3,4-tetrahydronaphthaline der allgemeinen Formel X mit Alkalicyanaten - bevorzugterweise Kaliumcyanat -vorzugsweise in wässerigen Reaktionsmedien Harnstoffderivate der allgemeinen Formel XVI verfügbar gemacht.

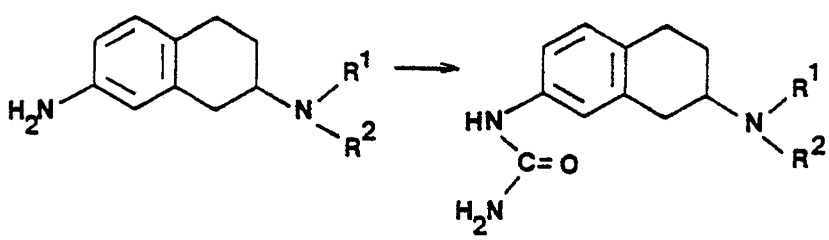

(X) (XVI)

Die hierzu benötigten Verfahren sind ebenfalls aus dem Stand der Technik wohlbekannt [C. Ferri, Reaktionen der organischen Synthese, Georg Thieme Verlag, Stuttgart 1978, S. 657 und zit. Lit.].

XII

Ausgehend von den 7-Acylamido-2-amino-1,2,3,4-tetrahydronaphthalinen der allgemeinen Formel VIII kann in einer weiteren Synthesevariante - unter Beibehaltung der ursprünglichen Acylgruppe - gewünschtenfalls eine weitere Alkylierung am Amido-Stickstoff durchgeführt werden, woraus 1,2,3,4-Tetrahydronaphthalin-Derivate der allgemeinen Formel XVII resultieren:

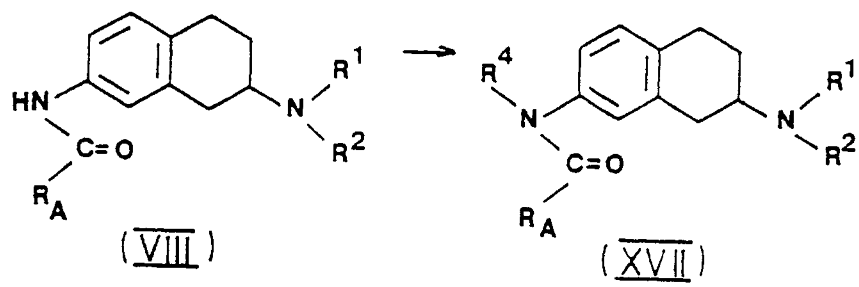

Zum Zweck der Substitution am Amido-Stickstoff setzt man hierbei im allgemeinen die Alkali-Derivate -bevorzugt die Natrium-Derivate - des entsprechenden Säureamids mit dem gewünschten Alkyl- oder Aralkylhalogenid um [G. Spielberger in Houben-Weyl, Methoden der organischen Chemie, Georg Thieme Verlag, Stuttgart 1957, S. 96; W.F. Fones, J. Org. Chem. 14 (1949) 1099; R.A.W. Johnstone, D.W. Payling und C. Thomas, J. Chem. Soc. [C] 1969, 2223].

Als Reaktionsmedien eignen sich hierzu Ether - wie Glykoldimethylether (Glyme), Diglykoldimethylether (Diglyme), Tetrahydrofuran - oder Sulfoxide - wie Dimethylsulfoxid - oder Säureamide, unter denen Dimethylformamid besonders bevorzugt wird.

Die Umsetzung - vorzugsweise mit Natriumhydrid als Base - wird in Abhängigkeit von dem verwendeten Lösungsmittel bevorzugt in einem Temperaturbereich von -10 °C bis 40 °C und besonders bevorzugt in einem Temperaturintervall von 0 °C bis 10 °C durchgeführt, während die Umsetzung des resultierenden Natriumacylamids in Abhängigkeit von der Reaktivität des eingesetzten Halogenorganyls in einem Temperaturbereich von 0 °C bis zum Siedepunkt der Reaktionsmischung und bei Verwendung von Dimethylformamid als Reaktionsmedium vorzugsweise bei Rückflußtemperatur der Reaktionsmischung durchgeführt wird.

XIII

In einer weiteren Synthesevariante wird die Säureamidfunktion zu dem entsprechenden sekundären bzw. tertiären Amin reduziert, woraus Diamine der allgemeinen Formel XVIII resultieren:

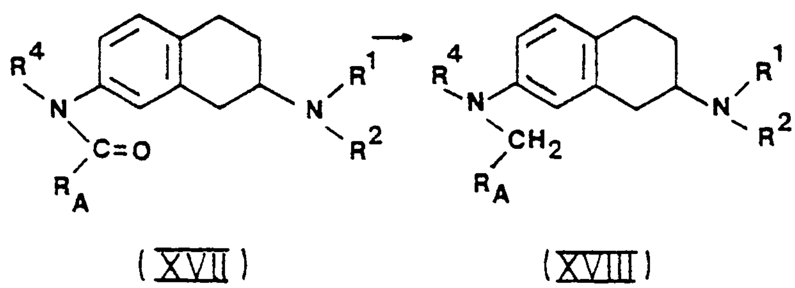

Derartige Reduktionen von Säureamiden sind aus dem Stand der Technik bekannt und können auf dem Wege der elektrolytischen Reduktion, durch Reduktion mit Alkalimetallen sowie durch katalytische Reduktion [R. Schröter in Houben-Weyl, Methoden der organischen Chemie, Band XI/1, Georg Thieme Verlag, Stuttgart 1957, S. 574] oder mit Diboran bzw. Borwasserstoff-Derivaten [J. Fuhrhop und G. Penzlin, Organic Synthesis -Concepts - Methods - Starting Materials, VCH-Verlagsgesellschaft, Weinheim 1986, S. 90] erfolgen.

Bevorzugt wird die Reduktion mit komplexen Hydriden, wie Alkalibor- oder Alkalialuminiumhydriden bzw. mit deren geeigneten Derivaten - ggf. in Gegenwart eines Katalysators - [N.G. Gaylord, Reduction with Complex Metal Hydrides, Wiley New York 1965; A. Hájos, Complex Hydrides, Elsevier New York 1979; V. Bažant, M. Čapka, M. Černy, V. Chvalovsky, K. Kochloefl, M. Kraus und J. Malek, Tetrahedron Lett. 9 -(1968) 3303] wobei Lithiumaluminiumhydrid besonders bevorzugt wird.

Als Reaktionsmedien eignen sich hierbei alle inerten organischen Lösungsmittel, die sich unter den gegebenen Reaktionsbedingungen nicht verändern. Hierzu gehören bevorzugt Ether wie z.B. tert.-Butylmethylether, Di-n-butylether, Glykoldimethylether (Glyme), Diglykoldimethylether (Diglyme), Tetrahydrofuran, Dioxan und besonders bevorzugt Diethylether.

Alle allgemeinen Formeln II bis XVIII fallen unter den Anspruch der allgemeinen Formel I.

Die erfindungsgemäßen Wirkstoffe werden in Einzeldosen von 1 bis 300 mg, vorzugsweise 10 bis 150 mg (oral) bzw. 1 bis 20 mg (parental) verabreicht.

Die erfindungsgemäßen Wirkstoffe können in die üblichen galenischen Anwendungsformen, wie Tabletten, Dragées, Lösungen, Emulsionen, Pulver, Kapseln oder Depotformen gebracht werden, wobei zu deren Herstellung die üblichen pharmazeutischen Hilfsstoffe sowie die üblichen Fertigungsmethoden herangezogen werden können. Entsprechende Tabletten können beispielsweise durch Mischen der Wirkstoffe mit bekannten Hilfsstoffen, beispielsweise inerten Verdünnungsmitteln, wie Calciumcarbonat, Calciumphosphat oder Milchzucker, Sprengmitteln, wie Maisstärke oder Alginsäure, Bindemitteln, wie Stärke oder Gelatine, Schmiermitteln, wie Magnesiumstearat oder Talk und/oder Mitteln zur Erzielung eines Depoteffektes, wie Carboxymethylcellulose, Celluloseacetphthalat, oder Polyvinylacetat erhalten werden.

Die Tabletten können auch aus mehreren Schichten bestehen. Entsprechend können Dragées durch Überziehen von analog den Tabletten hergestellten Kernen mit den überlicherweise in Dragéeüberzügen verwendeten Mitteln, beispielsweise Kollidon oder Schellack, Gummi arabicum, Talk, Titandioxid oder Zucker, hergestellt werden. Zur Erzielung eines Depoteffekts oder zur Vermeidung von Inkompatibilitäten kann der Kern auch aus mehreren Schichten bestehen. Desgleichen kann auch die Dragéehülle zur Erzielung eines Depoteffektes aus mehreren Schichten bestehen, wobei die oben bei den Tabletten erwähnten Hilfsstoffe verwendet werden können.

Säfte der erfindungsgemäßen Wirkstoffe bzw. Wirkstoffkombinationen können zusätzlich noch ein Süßungsmittel, wie Saccharin, Cyclamat, Glycerin oder Zucker, sowie ein geschmackverbesserndes Mittel, z.B. Aromastoffe, wie Vanillin oder Orangenextrakt, enthalten. Sie können außerdem Suspendierhilfsstoffe oder Dickungsmittel, wie Natriumcarboxymethyl-cellulose Netzmittel, beispielsweise Kondensationsprodukte von Fettalkoholen mit Ethylenoxid, oder Schutzstoffe, wie p-Hydroxybenzoate, enthalten.

Injektionslösungen werden in üblicher Weise, z.B. unter Zusatz von Konservierungsmitteln, wie p-Hydroxybenzoaten, oder Stabilisatoren, wie Komplexonen, hergestellt und in Injektionsflaschen oder Ampullen abgefüllt.

Die Wirkstoffe bzw. Wirkstoffkombinationen enthaltenden Kapseln können beispielsweise in der Weise hergestellt werden, daß man die Wirkstoffe mit inerten Trägern, wie Milchzucker oder Sorbit, mischt und in Gelatinekapseln einkapselt.

Geeignete Zäpfchen lassen sich beispielsweise durch Vermischen der dafür vorgesehenen Wirkstoffe bzw. Wirkstoffkombinationen mit den üblichen Trägermitteln, wie Neutralfetten oder Polyäthylenglykol bzw. dessen Derivaten, herstellen.

Formulierungsbeispiele

| 1. Tabletten | |
|---|---|
| Verbindung nach Beispiel 11 | 10,0 mg |
| Maisstärke | 99,0 mg |
| sek. Calciumphosphat | 140,0 mg |
| Magnesiumstearat | 1,0 mg |
| | 250,0 mg |

Die Bestandteile werden in üblicher Weise zu Tabletten von 250 mg Gewicht verarbeitet.

| Kapseln | |
|---|---|
| Verbindung nach Beispiel 11 | 150,0 mg |
| Maisstärke | 150,0 mg |
| | 300,0 mg |

Die fein gepulverten Komponenten werden intensiv vermischt. Jeweils 300 mg der Mischung werden in übliche Gelatinekapseln abgefüllt.

Die nachfolgenden Herstellungsbeispiele erläutern die Erfindung, ohne sie einzuschränken.

Das in den Herstellungsbeispielen vor den Substanzbezeichnungen angegebene Praefix (+)/(-) soll bedeuten, daß diese Synthesen sowohl mit den linksdrehenden als auch mit den rechtsdrehenden optischen Isomeren bzw. mit deren Mischung bzw. dem entsprechenden Racemat durchführbar sind. Alle Ausbeute-Angaben beziehen sich auf % der Theorie.

## Beispiel 1

Trennung der enantiomeren 2,7-Diamino-1,2,3,4-tetrahydronaphthaline

81,0 g (0,5 mol) 2,7-Diamino-1,2,3,4-tetrahydronaphthalin werden in 1 l einer Methanol/Wasser-Mischung (95:5) gelöst und auf 50 °C erwärmt. Bei dieser Temperatur wird eine Lösung von 37,5 g (0,25 mol) L-(+)-Weinsäure in 200 ml Methanol zugegeben. Die nach kurzer Zeit ausgefallenen Kristalle werden abgesaugt, dreimal mit jeweils 300 ml Methanol/Wasser-Mischung (95:5) ausgekocht und einmal aus Wasser umkristallisiert. Anschließend suspendiert man das Tartrat in 70 ml Wasser, versetzt mit 33 g (0,6 mol) Kaliumhydroxyd und extrahiert jeweils zweimal mit 70 ml Tetrahydrofuran. Nach dem Trocknen der organischen Phase mit Magnesiumsulfat und dem Abfiltrieren des Trockenmittels wird das Lösungsmittel i. Vak. entfernt und der Rückstand unter einem verminderten Druck von 0,08 hPa destilliert.

Man isoliert das (+)-2,7-Diamino-1,2,3,4-tetrahydronaphthalin als farblose Kristalle.
Ausbeute: 9,7 g (12 %)
Schmp. : 33 - 34 °C
Sdp. : 108 - 110 °C

Das (-)-Enantiomer wird auf analogem Wege unter Verwendung von R-(-)-Weinsäure erhalten.
Ausbeute: 9,8 g (12 %)
Schmp. : 33 - 34 °C
Sdp. : 108 - 110 °C

In den folgenden Beispielen wird zur Darstellung der enantiomerenreinen Verbindungen jeweils von (+)-2,7-Diamino-1,2,3,4-tetrahydronaphthalin bzw. von (-)-2,7-Diamino-1,2,3,4-tetrahydronaphthalin ausgegangen.

## Beispiel 2

(+)/(-)-7-Amino-2-tert.-butoxycarbonylamido-1,2,3,4-tetrahydronaphthalin

Zu einer Lösung von 20 g (0,12 mol) (+)/(-)-2,7-Diamino-1,2,3,4-tetrahydronaphthalin und 17,1 ml (0,12 mol) Triethylamin in 250 ml wasserfreiem Tetrahydrofuran wird bei -10 °C eine Lösung von 28,7 ml (0,12 mol) Pyrokohlensäure-di-tert.-butylester in 140 ml wasserfreiem Tetrahydrofuran zugetropft. Man rührt 30 Minuten bei -10 °C nach und läßt die Reaktionslösung anschließend auf Raumtemperatur erwärmen. Das Lösungsmittel wird i. Vak. abdestilliert und der Rückstand mit 500 ml Essigester versetzt und zweimal mit je 100 ml Wasser gewaschen. Anschließend wird die organische Phase mit Magnesiumsulfat getrocknet, das Lösungsmittel i. Vak. entfernt und der Rückstand aus Diethylether umkristallisiert.
Ausbeute: 25 g (80 %)
Schmp. : 119 - 121 °C

## Beispiel 3

(+)/(-)-7-Acetamido-2-tert.-butoxycarbonylamido-1,2,3,4-tetrahydronaphthalin

25 g (95 mmol) 7-Amino-2-tert.-butoxycarbonylamido-1,2,3,4-tetrahydronaphthalin und 14,5 ml (100 mmol) Triethylamin werden in 120 ml wasserfreiem Tetrahydrofuran gelöst und bei Raumtemperatur mit 9,9 ml (105 mmol) Essigsäureanhydrid nach und fügt anschließend 450 ml Eiswasser zu. Die ausgefallenen Kristalle werden abgesaugt und mit 200 ml Wasser, mit 35 ml Aceton und mit 60 ml Diethylether gewaschen und im Umlufttrockenschrank bis zur Gewichtskonstanz getrocknet.
Ausbeute: 25,9 g (93 %)
Schmp. : 193 - 195 °C

Beispiel 4

(+)/(-)-7-Acetamido-2-amino-1,2,3,4-tetrahydronaphthalin-Hydrochlorid

50 g (0,16 mol) 7-Acetamido-2-tert.-butoxycarbonylamido-1,2,3,4-tetrahydronaphthalin werden in 300 ml Ethanol suspendiert. Man leitet etwa 10 Minuten einen kräftigen HCl-Gasstrom durch die Suspension, wobei man die Temperatur auf 70 °C ansteigen läßt. Man läßt auf Raumtemperatur abkühlen, saugt die ausgefallenen Kristalle ab, wäscht je einmal mit 100 ml Aceton und 100 ml Diethylether und trocknet im Umlufttrokkenschrank zunächst bei Raumtemperatur, dann bei 80 °C unter Normaldruck bis zu Gewichtskonstanz.
Ausbeute: 36 g (91 %)
Schmp. : 260 °C

Beispiel 5

(+)/(-)-7-Acetamido-2-propylamino-1,2,3,4-tetrahydronaphthalin-Hydrochlorid

(Alkylierung)

83,8 g (0,41 mol) (+)/(-)-7-Acetamido-2-amino-1,2,3,4-tetrahydronaphthalin und 75,6 g (0,61 mol) Propylbromid werden in 900 ml Tetrahydrofuran gelöst, mit 84 g (0,84 mol) Kaliumhydrogencarbonat versetzt und 7 Stunden auf Rückflußtemperatur erhitzt. Anschließend wird mit weiteren 75,6 g (0,61 mol) Propylbromid versetzt und erneut 7 Stunden zum Sieden erhitzt. Man läßt auf Raumtemperatur abkühlen, filtriert und engt das Filtrat i. Vak. ein, versetzt mit 500 ml Essigsäureethylester und extrahiert mit 500 ml Wasser. Die organische Phase wird mit Magnesiumsulfat getrocknet, nach dem Abfiltrieren des Trockenmittels i. Vak. eingeengt und der verbleibende Rückstand an Kieselgel (Korngröße 0,063 bis 0,2 mm) mit einer Mischung aus Methylenchlorid und Methanol (80:20) chromatographiert. Nach der chromatographischen Isolierung, dem Einengen des Eluates: i. Vak. und Umsetzung des Rückstandes mit einer Lösung von Chlorwasserstoff in Diethylether wird die Titelverbindung als Hydrochlorid gefällt, abfiltriert und im Umlufttrockenschrank bis zur Gewichtskonstanz getrocknet.
Ausbeute: 43,4 g (38 %)
Schmp. : 171 °C

Analog zu Beispiel 5 werden die Hydrochloride der folgenden Verbindungen hergestellt, wobei zur Darstellung der dialkylierten 7-Acetamido-2-diallyl-bzw. 7-Acetamido-2-alkylaralkyl-1,2,3,4-tetrahydronaphthalin-Derivate Dimethylformamid als Reaktionsmedium eingesetzt wird:

Tabelle 5

| $R^1$ | $R^2$ | $R^3$ | $R^4$ | Schmp.: |
|---|---|---|---|---|
| H | $C_2H_5$ | Acetyl | H | 212-214°C |
| H | 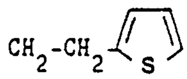 | Acetyl | H | 233-234°C |
| H | 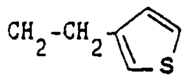 | Acetyl | H | 231-233°C |
| Butyl | $(CH_2)_3-CH_3$ | Acetyl | H | 178-180°C |
| Propyl | $(CH_2)_3-CH_3$ | Acetyl | H | 226-228°C |
| Propyl | 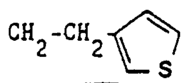 | Acetyl | H | ab 95°C |
| Propyl | 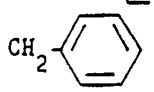 | Acetyl | H | 103-106°C |
| Propyl | 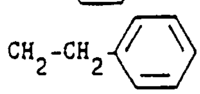 | Acetyl | H | 117-120°C |
| Propyl | 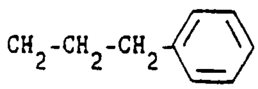 | Acetyl | H | 106-109°C |
| Propyl | $CH_2-CH=CH_2$ | Acetyl | H | 257-258°C |
| Propyl | 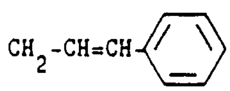 | Acetyl | H | ab 135°C |
| Allyl | $CH_2-CH=CH_2$ | Acetyl | H | 228-230°C |
| Propyl | $CH_2-C\equiv CH$ | Acetyl | H | 231-233°C |
| Propyl | 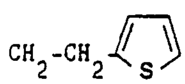 | Acetyl | H | ab 105°C |
| Propyl | 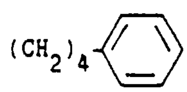 | Acetyl | H | ab 82°C |
| Propyl | 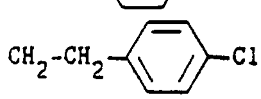 | Acetyl | H | 107-110°C[1)] |
| Propyl | 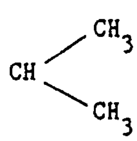 | Acetyl | H | 104-106°C[1)] |

| $R^1$ | $R^2$ | $R^3$ | $R^4$ | Schmp.: |
|---|---|---|---|---|
| Propyl | $CH_2$- 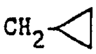 | Acetyl | H | 105-107°C 1) |
| $(CH_2)_3$- | $(CH_2)_3$- 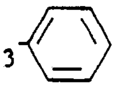 | Acetyl | H | ab 94°C |
| Propyl | $CH_2$-$CH_2$-$CH_2$- 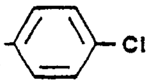 | Acetyl | H | ab 106°C |

Tabelle 6

$R^1$ = Propyl, $R^3$ = Acetyl, $R^4$ = H

| $R^2$ | $[\alpha]_D 20$ 2) | Schmp.: |
|---|---|---|
| $(CH_2)_3$-$CH_3$ | - 79,2° | 208-210°C |
| $(CH_2)_3$-$CH_3$ | + 78,5° | 208-210°C |
| $CH_2$-$CH_2$- 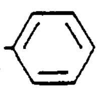 | - 54,7° | ab 115°C |
| $CH_2$-$CH_2$- 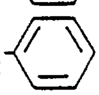 | + 55,3° | ab 115°C |
| $CH_2$-$CH_2$-$CH_2$- 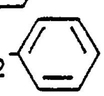 | - 62,9° | ab 92°C |
| $CH_2$-$CH_2$-$CH_2$- 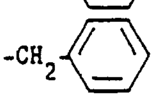 | + 63,7° | ab 88°C |
| $CH_2$-CH=CH- 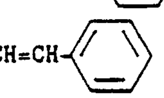 | -110,3° | ab 125°C |
| $CH_2$-CH=CH- 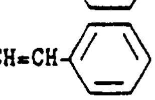 | +103,4° | 84-86°C 1) |

1) Schmelzpunkt der freien Base
2) in Methanol c = g.dm/100 ml

Beispiel 6

(+)/(-)-7-Acetamido-2-[(3-phenylallyl)amino]-1,2,3,4-tetrahydronaphthalin-Hydrochlorid

(reduktive Aminierung mit Natriumtetrahydridoboranat [$NaBH_4$])

4,8 g (23 mmol) (+)/(-)-7-Acetamido-2-amino-1,2,3,4-tetrahydronaphthalin und 3 g (23 mmol) Zimtaldehyd werden in 50 ml Dimethylformamid gelöst und eine Stunde lang bei 100°C gerührt. Im Anschluß daran wird das Lösungsmittel i.Vak. abdestilliert und der Rückstand mit 50 ml Ethanol und 1 g (26 mmol) Natriumtetrahydridoboranat ($NaBH_4$) versetzt. Man rührt 1 Stunde bei 30-40°C nach, entfernt anschließend das Lösungsmittel i. Vak.. Der Rückstand wird mit 200 ml Essigester versetzt, zweimal mit jeweils 100 ml Wasser gewaschen und einmal mit 200 ml 2N HCl extrahiert. Der wässerige Extrakt wird darauf mit konz. wässeriger Ammoniaklösung alkalisch gestellt und zweimal mit jeweils 100 ml Essigester extrahiert; die organische Phase wird mit Magnesiumsulfat getrocknet und nach dem Abfiltrieren des Trockenmittels i. Vak. eingeengt. Der verbleibende Rückstand wird in Ethanol gelöst und zur Fällung des Hydrochlorides mit etherischer Chlorwasserstoff-Lösung versetzt. Die Kristalle werden abgesaugt und im Umlufttrockenschrank bis zur Gewichtskonstanz getrocknet.
Ausbeute: 5 g (60 %)
Schmp.: 253 - 255°C

Analog zu Beispiel 6 wird das (+)- und (-)-7-Acetamido-2-benzylamino-1,2,3,4-tetrahydronaphthalin hergestellt.
Ausbeute: 86 %
Schmp.: 233 - 234°C

Beispiel 7

(+)/(-)-7-Acetamido-2-N,N-dimethylamino-1,2,3,4-tetrahydronaphthalin-Hydrochlorid

(reduktive Aminierung mit Wasserstoff)

6,5 g (32 mmol) 7-Acetamido-2-amino-1,2,3,4-tetrahydronaphthalin und 22 g (500 mmol) Acetaldehyd werden in 110 ml Methanol gelöst und mit 2,2 g Palladium / Kohle (10 % Pd) versetzt. Man hydriert 18 Stunden im Autoklaven bei 30°C und einem Wasserstoffdruck von 500 kPa. Anschließend wird über Kieselgel abgesaugt und das Lösungsmittel i. Vak. abdestilliert. Der Rückstand wird anschließend mit 150 ml Diethylether versetzt und zweimal mit jeweils 60 ml Wasser gewaschen. Die organische Phase wird mit Magnesiumsulfat getrocknet, das Lösungsmittel nach dem Abfiltrieren des Trockenmittels i. Vak. entfernt und der verbleibende Rückstand an Kieselgel (Korngröße 0,063-0,2 mm) chromatographiert (Fließmittel: Essigester / Methanol; 80:20). Nach der chromatographischen Isolierung wird das Eluat eingeengt und das (+)/(-)-Acetamido-2-N,N-diethylamino-1,2,3,4-tetrahydronaphthalin mit etherischer Salzsäure in das Hydrochlorid überführt. Die Kristalle werden abgesaugt und im Umlufttrockenschrank bis zur Gewichtskonstanz getrocknet.
Ausbeute: 1,7 g (18 %)
Schmp.: > 265°C

Beispiel 8

(+)/(-)-7-Acetamido-2-(N-allyl-N-methyl)amino-1,2,3,4-tetrahydronaphthalin-Hydrochlorid

(Leukart-Wallach-Aminierung

2,0 g (8,2 mmol) (+)/(-)-7-Acetamido-2-allylamino-1,2,3,4-tetrahydronaphthalin werden in 40 ml 99 proz. Ameisensäure gelöst und mit 10 ml wasseriger 30 proz. Formalinlösung versetzt. Man erhitzt 1,5 Stunden auf Rückflußtemperatur, engt i. Vak. ein, versetzt den Rückstand mit Eis und stellt mit konz. wässeriger Ammoniaklösung alkalisch.

Im Anschluß daran wird zweimal mit jeweils 100 ml Essigester extrahiert, die vereinigten organischen Phasen werden mit Magnesiumsulfat getrocknet und das Lösungsmittel nach dem Abfiltrieren des Trockenmittels i. Vak. abdestilliert. Der verbleibende Rückstand wird an Kieselgel (Korngröße 0,063-0,2 mm) chromatographiert (Fließmittel: Methylenchlorid / Methanol; 80:20). Nach der chromatographischen Isolierung des (+)/(-)-7-Acetamino-2-(N-allyl-N-methyl)amino-1,2,3,4-tetrahydronaphthalins wird das Eluat eingeengt und die Base mit etherischer Salzsäure in das Hydrochlorid überführt. Die Kristalle werden abgesaugt und im Umlufttrockenschrank bis zur Gewichtskonstanz getrocknet.
Ausbeute: 1,8 (75 %)
Schmp. : 169 - 171 °C

Analog zu Beispiel 8 wird das (+)/(-)-7-Acetamido-2-[N-methyl-N-(3-phenylallyl)]amino-1,2,3,4-tetrahydronaphthalin dargestellt:
Asubeute: 35 %
Schmp. : 249 - 251 °C

## Beispiel 9

(+)/(-)-7-Amino-2-ethylamino-1,2,3,4-tetrahydronaphthalin-Hydrochlorid

1,6 g (5,9 mmol) (+)/(-)-7-Acetamido-2-ethylamino-1,2,3,4-tetrahydronaphthalin-Hydrochlorid werden mit 40 ml 2N HCl 2 Stunden auf Rückflußtemperatur erhitzt. Anschließend wird die Lösung i. Vak. eingeengt, der Rückstand mit 20 ml Ethanol versetzt, die Kristalle werden abgesaugt, mit Ethanol gewaschen und im Umlufttrockenschrank bis zum Gewichtskonstanz getrocknet.
Ausbeute: 1,3 g (84 %)
Schmp. : >260 °C

Tabelle 7

Analog Beispiel 9 wurden die Hydrochloride der folgenden Verbindungen dargestellt

| $R^1$ | $R^2$ | $R^3$ | $R^4$ | Schmp.: |
|---|---|---|---|---|
| H | $CH_2$-$CH_2$-$CH_3$ | H | H | 198-199°C |
| H | $CH_2$- 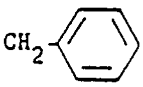 | H | H | ab 150°C [1)] |
| H | $CH_2$-$CH_2$- 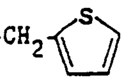 | H | H | ab 258°C [1)] |
| Ethyl | $C_2H_5$ | H | H | 153-155°C |
| Butyl | $-(CH_2)_3-CH_3$ | H | H | 154-156°C |
| Propyl | $CH_3$ | H | H | 178-180°C |
| Propyl | $-(CH_2)_3-CH_3$ | H | H | 152-154°C |
| Propyl | $CH_2$- 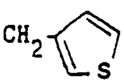 | H | H | ab 180°C |
| Propyl | $CH_2$- 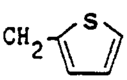 | H | H | ab 180°C [1)] |
| Propyl | $CH_2$-$CH_2$- 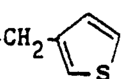 | H | H | 187-190°C [1)] |
| Propyl | $CH_2$- 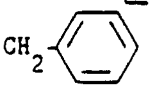 | H | H | 165-168°C |
| Methyl | $CH_2$- 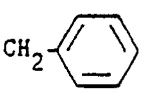 | H | H | 210-212°C [1)] |
| Propyl | $CH_2$-$CH_2$- 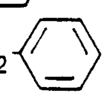 | H | H | 145-148°C |
| Methyl | $CH_2$-$CH_2$- 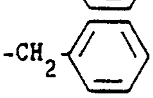 | H | H | 125-128°C |
| Propyl | $CH_2$-$CH_2$-$CH_2$- 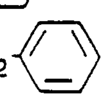 | H | H | 105-108°C |
| Propyl | $CH_2$-$CH$=$CH_2$ | H | H | 152-154°C |
| Methyl | $CH_2$-$CH$=$CH_2$ | H | H | 79-82°C |
| Allyl | $CH_2$-$CH$=$CH_2$ | H | H | 94-97°C |
| H | $CH_3$ | H | H | >260°C [1)] |

| $R^1$ | $R^2$ | $R^3$ | $R^4$ | Schmp.: |
|---|---|---|---|---|
| Propyl | $CH_2$-$CH_2$-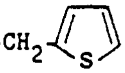 | H | H | ab 110°C [1)] |
| Propyl | $CH_2$-$CH_2$-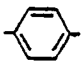-Cl | H | H | ab 165°C [1)] |
| Propyl | $(CH_2)_4$-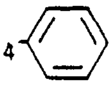 | H | H | ab 135°C [1)] |
| Propyl | $CH_2$-C≡CH | H | H | 70-72°C |
| Propyl | $CH_2$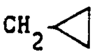 | H | H | 91-93°C |
| Propyl | $CH_2$-CH=CH-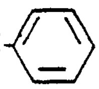 | H | H | 182-184°C[1)] |
| $(CH_2)_3$-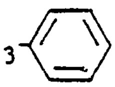 | $(CH_2)_3$-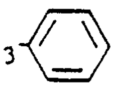 | H | H | ab 140°C [1)] |

[1)]Dihydrochlorid

Tabelle 8

Analog Beispiel 9 wurden die Dihydrochloride folgender Verbindungen hergestellt:

$R^1$ = Propyl, $R^3$ = H, $R^4$ = H

| $R^2$ | $[\alpha]_D 20$ 1) | Schmp.: |
|---|---|---|
| $-(CH_2)_3-CH_3$ | - 66,4° | ab 150°C |
| $-(CH_2)_3-CH_3$ | + 66,4° | ab 151°C |
| $CH_2-CH_2-$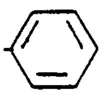 | - 31,6° | ab 146°C |
| $CH_2-CH_2-$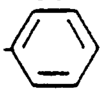 | + 51,1° | ab 142°C |
| $CH_2-CH_2-CH_2-$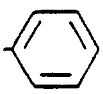 | - 55,3° | ab 158°C |
| $CH_2-CH_2-CH_2-$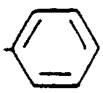 | + 54,3° | ab 157°C |
| $CH_2-CH=CH-$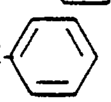 | - 74,7° | ab 180°C |
| $CH_2-CH=CH-$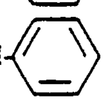 | + 88,3° | ab 150°C 2) |

1) in Methanol c = g.100 $ml^{-1}$.dm
2) Hydrochlorid

## Beispiel 10

(+)/(-)-2-Dipropylamino-7-methansulfonamido-1,2,3,4-tetrahydronaphthalin-Hydrochlorid

1,7 g (6,9 mmol) (+)/(-)-7-Amino-2-dipropylamino-1,2,3,4-tetrahydronaphthalin und 1,1 ml (7,6 mmol) Triethylamin werden in 25 ml Tetrahydrofuran gelöst. Zu dieser Lösung tropft man bei 15 °C 0,9 g (7,6 mmol) Mesylchlorid und läßt anschließend noch 1 Stunde bei Raumtemperatur nachrühren. Im Anschluß daran destilliert man das Lösungsmittel unter vermindertem Druck ab, versetzt den Rückstand mit 200 ml Essigester und wäscht zweimal mit jeweils 80 ml Wasser. Die organische Phase wird mit Magnesiumsulfat getrocknet und das Lösungsmittel nach dem Abfiltrieren des Trockenmittels i. Vak. entfernt. Die zurückbleibende Base wird mit etherischer Salzsäure in das kristalline Hydrochlorid überführt, welches im Umlufttrokkenschrank bis zur Gewichtskonstanz getrocknet wird.
Ausbeute: 1,5 g (60 %)
Schmp. : 256 - 257 ° C

Analog zu Beispiel 10 wird unter Verwendung von Trifluoressigsäureanhydrid und bei 8-stündigem Erwärmen auf Rückflußtemperatur das (+)/(-)-2-(N-Phenethyl-N-propyl)amino-7-trifluoracetamido-1,2,3,4-tetrahydronaphthalin dargestellt:
Schmp.: ab 108 ° C

Beispiel 11

(+)/(-)-7-Formylamido-2-(N-phenethyl-N-propyl)amino-1,2,3,4-tetrahydronapthalin-Hydrochlorid

1,5 g (4,9 mmol) (+)/(-)-7-Amino-2-(N-phenethyl-N-propyl)amino-1,2,3,4-tetrahydronaphthalin werden in 20 ml Ameisensäuremethylester gelöst und im Autoklaven 16 Stunden lang auf 120 ° C erhitzt. Nach dem Abkühlen wird das Lösungsmittel i. Vak. abdestilliert, der Rückstand mit jeweils 50 ml Essigester und Wasser versetzt und die wässerige Phase mit Kaliumcarbonat alkalisch gestellt. Man separiert die beiden Phasen und extrahiert die wässerige Phase noch zweimal mit jeweils 50 ml Essigester. Die vereinigten organischen Extrakte werden mit Magnesiumsulfat getrocknet und nach dem Abfiltrieren des Trockenmittels im Vakuum eingeengt. Anschließend chromatographiert man den verbleibenden Rückstand an Aluminiumoxid ($Al_2O_3$; N II-III) mit einer Mischung Cyclohexan / Essigester (1:1). Die auf diese Weise isolierte Base wird i. Vak. vom Lösungsmittel befreit und mit etherischer Salzsäure in das Hydrochlorid überführt.
Ausbeute: 1,3 g (71 %)
Schmp. : ab 98 ° C

Beispiel 12

(+)/(-)-7-Carbamoylamido-2-N,N-dipropylamino-1,2,3,4-tetrahydronapthalin

1,4 g (5,7 mmol) (+)/(-)-7-Amino-2-N,N-dipropylamino-1,2,3,4-tetrahydronaphthalin werden in 20 ml Wasser gelöst, mit 1,0 g Kaliumcyanat versetzt und 15 Minuten bei 80 ° C gerührt. Nach dem Abkühlen fügt man 10 ml konz. wässerige Ammoniaklösung zu und extrahiert zweimal mit jeweils 50 ml Essigester. Die vereinigten organischen Extrakte werden mit 30 ml Wasser gewaschen, anschließend mit Magnesiumsulfat getrocknet, nach dem Abfiltrieren des Trockenmittels und i. Vak. eingeengt. Der Rückstand wird aus Petrolether kristallisiert, und die Kristalle im Umlufttrockenschrank bis zur Gewichtskonstanz getrocknet.
Ausbeute: 1,1 g (67 %)
Schmp. : 139 - 141 ° C

Beispiel 13

(+)/(-)-2-N,N-Dipropylamino-7-(N-methyl-acetamido)-1,2,3,4-tetrahydronaphthalin-Hydrochlorid

Zu einer Suspension von 0,6 g (25 mmol) Natriumhydrid in 35 ml Dimethylformamid wird unter Eiskühlung eine Lösung von 5,5 g (19 mmol) (+)/(-)-7-Acetamido-2-N,N dipropylamino-1,2,3,4-tetrahydronaphthalin in 30 ml Dimethylformamid zugetropft. Man läßt ca. 30 min. nachrühren, bis keine Wasserstoffentwicklung mehr zu beobachten ist. Anschließend werden 1,4 ml (22 mmol) Methyliodid zugetropft. Danach wird die Reaktionsmischung 4 Stunden lang auf Rückflußtemperatur erhitzt. Man destilliert das Lösungsmittel i. Vak. ab, versetzt mit jeweils 100 ml Essigester und Wasser, trennt die organische Phase ab und extrahiert die wässerige Phase zweimal mit jeweils 50 ml Essigester. Die vereinigten organischen Extrakte werden mit Magnesiumsulfat getrocknet, das Lösungsmittel wird nach dem Abfiltrieren des Trockenmittels i.

Vak. abdestilliert und der verbleibende Rückstand an Kieselgel (Korngröße 0,063-0,2 mm) mit einer Essigester Cyclohexan-Mischung (1:1) chromatographiert Die auf diese Weise isolierte Fraktion des (+)/(-)-2-Dipropyl-amino-7-(N-methyl-acetamido-1,2,3,4-tetrahydronaphthalins wird in i. Vak. eingeengt und der Rückstand mit etherischer Salzsäure in das Hydrochlorid überführt. Die Kristalle werden im Umlufttrockenschrank getrocknet.
Schmp.: 208 - 211 °C

Analog zu Beispiel 13 wird das (+)/(-)-2-N,N-Dipropylamino-7-(N-methyl-formamido)-1,2,3,4-tetrahydronaphthalin dargestellt.
Schmp.: 61 - 62 °C

Beispiel 14

(+)/(-)-2-N,N-Dipropylamino-7-ethylamino-1,2,3,4-tetrahydronaphthalin-Hydrochlorid

0,7 g (18 mmol) Lithiumaluminiumhydrid werden in 40 ml wasserfreiem Ether vorgelegt. Unter Rühren wird eine Lösung von 5,9 g (18 mmol) 7-Acetamido-2-N,N-dipropylamino-1,2,3,4-tetrahydronapthalin in 50 ml wasserfreiem Diethylether bei ca. +10 °C über einen Zeitraum von 3 Stunden auf Rückflußtemperatur zugetropft. Anschließend erhitzt man die Reaktionsmischung, läßt anschließend auf ca. 5 °C abkühlen und versetzt die Suspension sukzessiv mit 0,7 ml Wasser, 2,1 ml 15 proz. Natronlauge und weiteren 0,7 ml Wasser. Der entstandene Niederschlag wird abgesaugt, dreimal mit jeweils 50 ml Ether gewaschen und die vereinigten etherischen Extrakte werden i. Vak. eingeengt. Der Rückstand wird an Kieselgel (Korngröße 0,063-0,2 mm) mit einer Mischung aus Cyclohexan und Essigester (1:1) chromatographiert. Die auf diese Weise isolierte Fraktion des (+)/(-)-2-N,N-Dipropylamino-7-ethylamino-1,2,3,4-tetrahydronaphthalins wird i. Vak. eingeengt und die zurückbleibende Base mit etherischer Salzsäure in das Hydrochlorid überführt. Die Kristalle werden im Umlufttrockenschrank bis zur Gewichtskonstanz getrocknet.
Ausbeute: 2,1 g (32 %)
Schmp. : ab 55 °C

Beispiel 15

(+)/(-)-7-Acetamido-2-methylamino-1,2,3,4-tetrahydronaphthalin-Hydrochlorid

(Reduktive Debenzylierung)

6,0 g (19 mmol) (+)/(-)-7-Acetamido-2-(N-benzyl-N-methyl)amino-1,2,3,4-tetrahydronaphthalin werden in 95 ml Methanol gelöst, mit 0,6 g Palladium auf Kohle (10 % Pd) versetzt und 5 Stunden lang bei 20 °C und einem Wasserstoffdruck von 500 kPa hydriert. Anschließend wird die Reaktionsmischung filtriert, das Lösungsmittel i. Vak. abdestilliert, der Rückstand mit etherischer Chlorwasserstoff-Lösung in das Hydrochlorid überführt, das aus Methanol umkristallisiert wird. Die Kristalle werden im Umlufttrockenschrank bis zur Gewichtskonstanz getrocknet.
Ausbeute: 1,5 g (29 %)
Schmp. : >265 °C

**Ansprüche**

1) 2,7-Diamino-1,2,3,4-tetrahydronaphthaline (2,7-Diaminotetraline) der allgemeinen Formel I

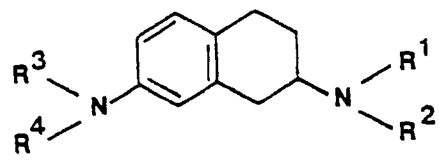

(I)

worin

$R^1$ Wasserstoff, $C_1$-$C_{12}$-Alkyl, $(CH_2)_a$-$R^5$, $CH_2$-CH = CH-$R^6$, Alkoxycarbonyl und

a eine Zahl 1, 2, 3 oder 4;

$R^2$ Wasserstoff, $C_1$-$C_{12}$-Alkyl, $(CH_2)_b$-$R^7$, Allyl, $CH_2$-CH = CH-$R^8$ und

b eine Zahl 1, 2, 3 oder 4;

$R^3$ Wasserstoff, $C_1$-$C_{12}$-Alkyl, Formyl, Acetyl, halogensubstituiertes Acetyl,

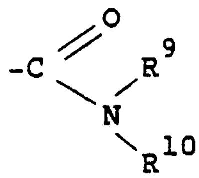 , 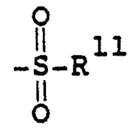 ;

$R^4$ Wasserstoff, $C_1$-$C_{12}$-Alkyl, $C_3$-$C_{12}$-Alkenyl, $C_3$-$C_{12}$-Alkinyl;

$R^5$ -CH = $CHR^{12}$, -C≡$CR^{13}$, -$CH_2$-O-$R^{14}$,

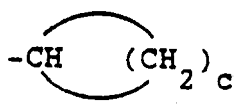 , 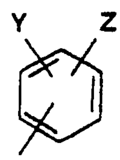 ,

Aryloxy, Aralkoxy, Alkylthio, Arylthio, Aralkylthio, Aryloxycarbonyl, Heteroaryl und substituiertes Heteroaryl, wie

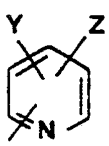 , 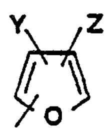 , 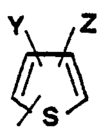 , 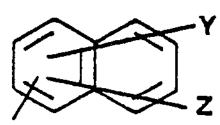 , 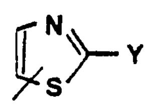 ,

und

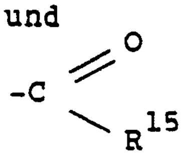 , 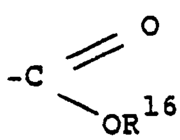 ,

worin

Y Wasserstoff, Halogen, Niederalkyl, Alkoxy, OH, $NH_2$, $NO_2$,

Z Wasserstoff, Halogen, Niederalkyl, Alkoxy, OH, $NH_2$, $NO_2$ und

c eine Zahl 1, 2, 3, 4 oder 5;

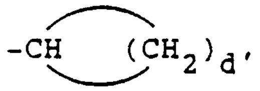, 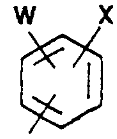, Aryloxy,

Aryloxy, Aralkoxy, Alkylthio, Aralkylthio, Arylthio, Aryloxycarbonyl, Heteroaryl und substituiertes Heteroaryl, wie

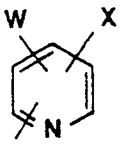, 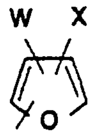, 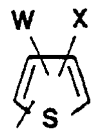, 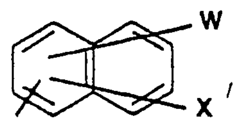, 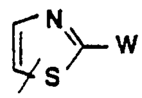,

und

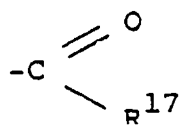, 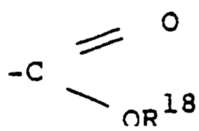,

worin

W Wasserstoff, Halogen, Niederalkyl, Alkoxy, OH, $NH_2$, $NO_2$,

X Wasserstoff, Halogen, Niederalkyl, Alkoxy, OH, $NH_2$, $NO_2$ und

d eine Zahl 1, 2, 3, 4 oder 5;

$R^7$ $-CH=CHR^{19}$, $-C\equiv CR^{20}$, $-CH_2-O-R^{21}$,

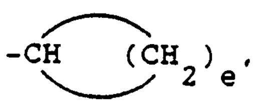, 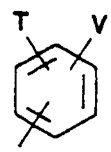,

Aryloxy, Aralkoxy, Alkylthio, Arylthio, Aralkylthio, Aryloxycarbonyl, Heteroaryl und substituiertes Heteroaryl, wie

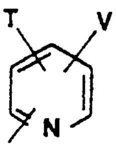, 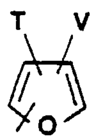, 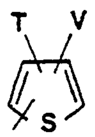, 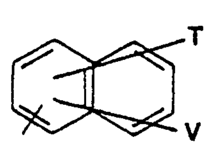, 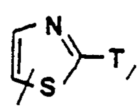,

und

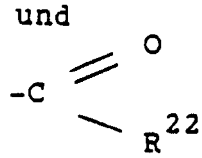, 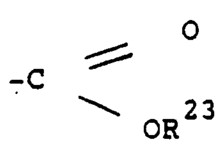,

worin

T Wasserstoff, Halogen, Niederalkyl, Alkoxy, OH, $NH_2$, $NO_2$,

V Wasserstoff, Halogen, Niederalkyl, Alkoxy, OH, $NH_2$, $NO_2$ und

e eine Zahl 1, 2, 3, 4 oder 5;

$R^8$ 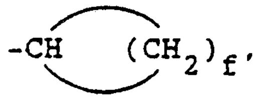 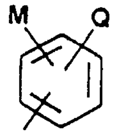 ,

Aryloxy, Aralkoxy, Arylthio, Alkylthio, Aralkylthio, Aryloxycarbonyl, Heteroaryl und substituiertes Heteroaryl, wie

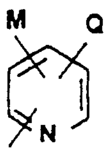 , 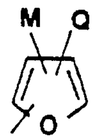 , 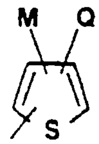 , 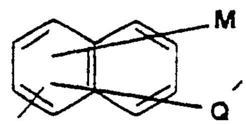 , 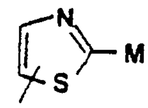

und

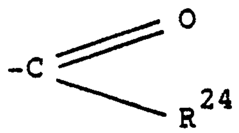 , 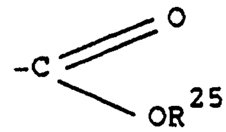 ,

worin
M Wasserstoff, Halogen, Niederalkyl, Alkoxy, OH, $NH_2$, $NO_2$,
Q Wasserstoff, Halogen, Niederalkyl, Alkoxy, OH, $NH_2$, $NO_2$ und
f eine Zahl 1, 2, 3, 4 oder 5;
$R^9$ Wasserstoff, Alkyl;
$R^{10}$ Wasserstoff, Alkyl;
$R^9$ und $R^{10}$ auch zusammen mit dem Stickstoffatom einen Heterocyclus, der noch ein weiteres Heteroatom - wie Stickstoff, Sauerstoff oder Schwefel - enthalten kann - z.B. einen Morpholin-, Piperidin- oder Piperaziniring -bilden können;
$R^{11}$ Alkyl, Aralkyl;
$R^{12}$ Wasserstoff, Alkyl, Aryl;
$R^{13}$ Wasserstoff, Alkyl;
$R^{14}$ Wasserstoff, Alkyl;
$R^{15}$ Alkyl, Aryl;
$R^{16}$ Alkyl, Aryl;
$R^{17}$ Alkyl, Aryl;
$R^{18}$ Alkyl, Aryl;
$R^{19}$ Wasserstoff, Alkyl, Aryl;
$R^{20}$ Wasserstoff, Alkyl, Aryl;
$R^{21}$ Wasserstoff, Alkyl;
$R^{22}$ Alkyl, Aryl;
$R^{23}$ Alkyl, Aryl;
$R^{24}$ Alkyl, Aryl;
$R^{25}$ Alkyl, Aryl;
bedeuten, deren Enantiomere, Racemate und Säureadditionssalze, wobei $R^1$, $R^2$, $R^3$ und $R^4$ nicht alle zusammen Wasserstoff und - im Falle $R^3$ und $R^4$ beide Wasserstoff oder $R^3$ Acetyl und $R^4$ Wasserstoff bedeuten - $R^1$ und $R^2$ nicht beide Methyl oder nicht beide Propyl bedeuten dürfen.

2) Verbindungen der allgemeinen Formel I, worin - mit den in Anspruch 1 gemachten Einschränkungen -

$R^1$ Wasserstoff, $C_1$-$C_6$-Alkyl, $(CH_2)_a$-$R^5$, $CH_2$-CH = CH-$R^6$, Alkoxycarbonyl und
a eine Zahl 1, 2, 3 oder 4;
$R^2$ Wasserstoff, $C_1$-$C_6$-Alkyl, $(CH_2)_b$-$R^7$, Allyl, $CH_2$-CH = CH-$R^8$ und
b eine Zahl 1, 2, 3 oder 4;
$R^3$ Wasserstoff, $C_1$-$C_6$-Alkyl, Formyl, Acetyl, halogensubstituiertes Acetyl,

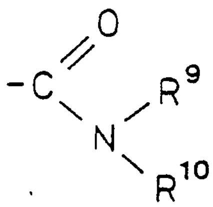 , 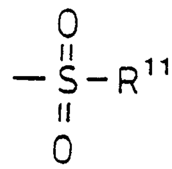 ;

$R^4$ Wasserstoff, $C_1$-$C_6$-Alkyl, $C_3$-$C_6$-Alkenyl, $C_3$-$C_6$-Alkinyl;
$R^5$, $R^6$, $R^7$ und $R^8$ die in Anspruch 1 angegebene Bedeutung haben;
$R^9$ und $R^{10}$ Wasserstoff oder Niederalkyl bedeuten, wobei $R^9$ und $R^{10}$ auch zusammen mit dem Stickstoffatom einen 5- oder 6-gliedrigen Heterocyclus - wie z.B. einen Morpholin-, Piperidin- oder Piperazinring -bilden können, in dem ein Kohlenstoffatom durch ein Heteroatom - wie z. B. Stickstoff, Sauerstoff oder Schwefel - ersetzt sein kann;
$R^{11}$ Niederalkyl oder Aralkyl bedeutet.

3) Verbindungen der allgemeinen Formel I, worin - mit den in Anspruch 1 gemachten Einschränkungen -

$R^1$ Wasserstoff, $C_1$-$C_4$-Alkyl, Allyl, tert.-Butoxycarbonyl;
$R^2$ Wasserstoff, $C_1$-$C_4$-Alkyl, $(CH_2)_b$-$R^7$, Allyl, $CH_2$-CH = CH-$R^8$,
b eine Zahl 1, 2, 3 oder 4;
$R^3$ Wasserstoff, Formyl, Acetyl, Trifluoracetyl,

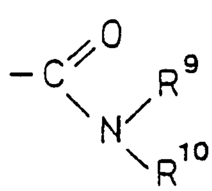 , 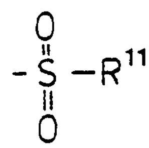

$R^4$ Wasserstoff, Methyl;
$R^7$ -CH = $CHR^{12}$, -C≡$CR^{13}$, Cyclopropyl, Phenyl,

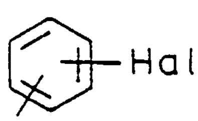 , 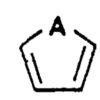 ,

worin Hal Chlor oder Brom und A Sauerstoff oder Schwefel;

$R^8$ 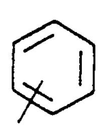 , 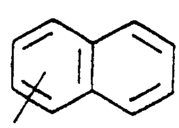 ;

$R^9$ und $R^{10}$ Wasserstoff oder Niederalkyl;
$R^{11}$ Niederalkyl;
$R^{12}$ Wasserstoff, Niederalkyl, Phenyl;
$R^{13}$ Wasserstoff oder Niederalkyl bedeutet.

4) (+) bzw. (-)-7-Acetamido-2-N-propyl-N-[2-(3-thienyl)ethyl]amino-1,2,3,4-tetrahydronaphthalin und deren Säureadditionssalze.

5) (+) bzw. (-)-7-Acetamido-2-(N-butyl-N-propyl)amino-1,2,3,4-tetrahydronaphthalin und deren Säureadditionssalze.

6) (+) bzw. (-)-7-Acetamido-2-(N-allyl-N-propyl)amino-1,2,3,4-tetrahydronaphthalin und deren Säureadditionssalze.

7) (+) bzw. (-)-7-Acetamido-2-(N-phenethyl-N-propyl)amino-1,2,3,4-tetrahydronaphthalin und deren Säureadditionssalze.

8) Pharmazeutische Zubereitung, gekennzeichnet durch den Gehalt an einer Verbindung nach einem der Ansprüche 1 bis 7 und (+)- bzw. (-)-2,7-Diamino-1,2,3,4-tetrahydronaphthalin, (+)- bzw. (-)-7-Aceamido-2-dimethylamino-1,2,3,4-tetrahydronaphtalin, (+)- bzw. (-)-7-Aceamido-2-dipropylamino-1,2,3,4-tetrahydronaphthalin, (+)- bzw. (-)-7-Amino-2-dimethylamino-1,2,3,4-tetrahydronaphthalin, (+)- bzw. (-)-7-Amino-2-dipropylamino-1,2,3,4-tetrahydronaphthalin
und deren Säureadditionssalze neben üblichen Hilfs-und Trägerstoffen.

9) Verwendung von Verbindungen nach Anspruch 1 bis 7 und (+)- bzw. (-)-2,7-Diamino-1,2,3,4-tetrahydronaphthalin, (+)- bzw. (-)-7-Aceamido-2-dimethylamino-1,2,3,4-tetrahydronaphtalin, (+)- bzw. (-)-7-Aceamido-2-dipropylamino-1,2,3,4-tetrahydronaphthalin, (+)- bzw. (-)-7-Amino-2-dimethylamino-1,2,3,4-tetrahydronaphthalin, (+)- bzw. (-)-7-Amino-2-dipropylamino-1,2,3,4-tetrahydronaphthalin
und deren Säureadditionssalze bei der Herstellung von pharmazeutischen Zubereitungen zur Behandlung der Schizophrenie sowie als Antipsychotika und als Sedativa.

10) Verfahren zum Herstellen von Verbindungen der allgemeinen Formel I,

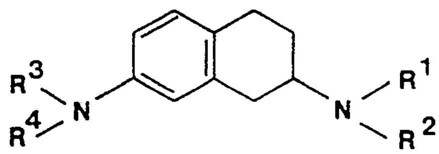
(I)

dadurch gekennzeichnet, daß man - im Falle

a) $R^1$ eine Alkoxycarbonylgruppe, $R^2$ und $R^3$ sowie $R^4$ Wasserstoff bedeuten - (+)- bzw. (-)-2,7-Diamino-1,2,3,4-tetrahydronaphthalin der allgemeinen Formel II bzw. III

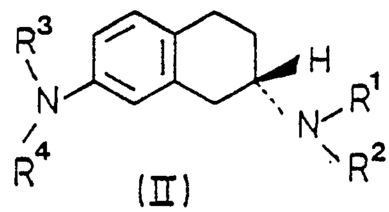
(II)

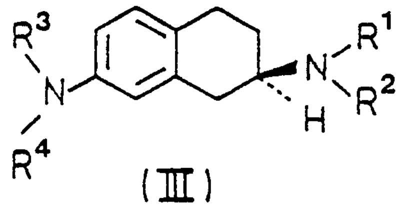
(III)

$R^1 = R^2 = R^3 = R^4 = H$

in an sich bekannter Weise in inerten Lösungsmitteln in Gegenwart einer Base mit einem Kohlensäure-Derivat der allgemeinen Formel XIX

$R_{Alk}-O-\underset{\underset{O}{\|}}{C}-A$ (XIX)

umsetzt, in welcher A für Halogen, $-O-\underset{\underset{O}{\|}}{C}-R_{Alk}$,

$-O-C_6H_5$, $-S-C_6H_5$, $-O-N=C(CN)C_6H_5$,

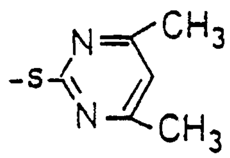

und $R_{Alk}$ für einen geradkettigen oder verzweigten Alkylrest steht;

b) $R^1$ eine Schutzgruppe, $R^2$ und $R^3$ sowie $R^4$ Wasserstoff bedeuten - (+)- bzw. (-)-2,7-Diamino-1,2,3,4-tetrahydronaphthalin der allgemeinen Formel II bzw. III

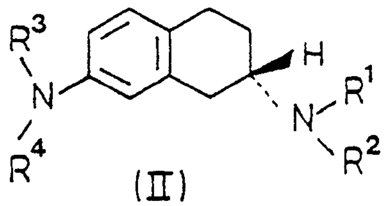

(II)

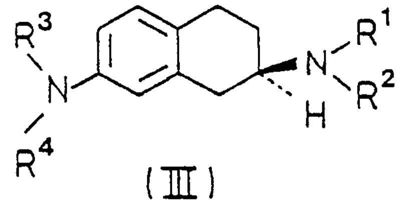

(III)

$R^1 = R^2 = R^3 = R^4 = H$

mit Verbindungen der allgemeinen Formel XX

$R_S$-B (XX)

in an sich bekannter Weise in einem inerten Lösungsmittel umsetzt, worin $R_S$ eine zum Schutz von Aminogruppen geeignete Schutzgruppe und B eine durch eine Aminofunktion substituierbare Austrittsgruppe bedeutet;

c) $R^1$ eine Schutzgruppe oder Alkoxycarbonylgruppe, $R^2$ und $R^4$ Wasserstoff und $R^3$ eine Acetylgruppe oder eine halogensubstituierte Acetylgruppe bedeutet -

2-Alkoxycarbonylamido-7-amino-1,2,3,4-tetrahydronaphthalin-Derivate der allgemeinen Formel VI

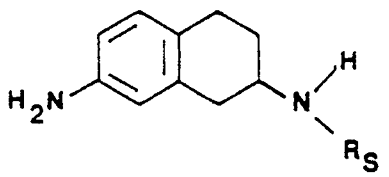

(IV)

mit einem Essigsäurederivat der allgemeinen Formel XXI

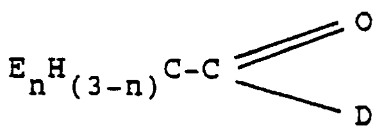

(XXI)

in einem inerten Lösungsmittel in Gegenwart einer Base umsetzt, wobei E für Halogen -untereinander gleich oder verschieden - n eine Zahl 0, 1, 2 oder 3 und D für Halogen oder für eine Aryloxy- oder Alkoxygruppe oder einen Rest der allgemeinen Formel XXII

$O-\overset{\overset{O}{||}}{C}-CE_nH_{(3-n)}$ (XXII)

steht, worin E und n die oben angegebene Bedeutung haben.

d) $R^1$, $R^2$ und $R^4$ Wasserstoff und $R^3$ - wie unter c) angegeben - eine Acetylgruppe oder eine mit einem oder mehreren Halogenatom(en) -untereinander gleich oder verschieden -substituierte Acetylgruppe bedeuten -

die Schutzgruppe $R_S$ aus 1,2,3,4-Tetrahydronaphthalin-Derivaten der allgemeinen Formel XXIII

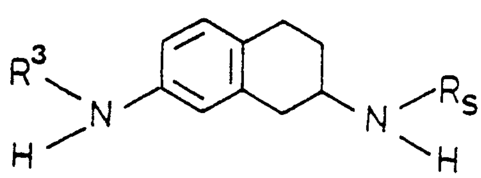

(XXIII)

in welcher $R_S$ für eine zum Schutz von Aminofunktion geeignete Gruppe steht unter den für die Abspaltung der jeweiligen Schutzgruppe $R_S$ an sich bekannten Reaktionsbedingungen abspaltet und durch Wasserstoff substituiert.

e) $R^1$ mit Ausnahme von Wasserstoff die in Anspruch 1 angegebene Bedeutung hat, $R^2$ und $R^4$ Wasserstoff und $R^3$ eine Acetylgruppe oder eine gegebenenfalls mit einem oder mehreren Halogenatom(en)

substituierte -untereinander gleich oder verschieden -Acetylgruppe bedeuten - 1,2,3,4-Tetrahydronaphthaline der allgemeinen Formel XXIV

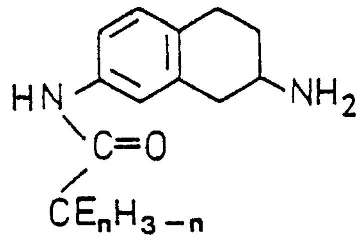

(XXIV)

- in der E für Halogen steht und n eine Zahl 0, 1, 2 oder 3 bedeutet wobei die Halogene untereinander gleich oder verschieden sein können - in einem inerten Lösungsmittel bzw. Lösungsmittelgemisch gegebenenfalls in Gegenwart einer Base mit Alkylierungsmitteln der allgemeinen Formel XXV

$G\text{-}R^1$ (XXV)

umsetzt, worin

G ein Halogen, einen Sulfat-, einen Tosylat-, einen Methansulfonat- oder einen Halogenmethansulfonatrest oder eine andere durch eine Aminofunktion substituierbare Gruppe bedeutet und

$R^1$ mit Ausnahme von Wasserstoff die in Anspruch 1 angegebene Bedeutung hat und gegebenenfalls zuvor geschützte funktionelle Gruppen in andere funktionelle Gruppen überführt und im Falle der Herstellung der verschiedenen Salze mit den entsprechenden Säuren umsetzt.

f) $R^1$ mit Ausnahme von Wasserstoff die in Anspruch 1 angegebene Bedeutung hat, $R^2$, $R^4$ Wasserstoff und $R^3$ einen Acetylgruppe oder gegebenenfalls eine mit einem oder mehreren Halogenatom-(en) - untereinander gleich oder verschieden - substituierte Acetylgruppe bedeuten - 1,2,3,4-Tetrahydronaphthalin-Derivate der allgemeinen Formel XXIV unter reduktiven Bedingungen in einem inerten Lösungsmittel gegebenenfalls in Anwesenheit eines Katalysators mit einer Carbonylverbindung der allgemeinen Formel XXVI

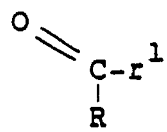

(XXVI)

umsetzt, wobei R Wasserstoff oder eine Alkylgruppe bedeutet und $-CHR\text{-}r^1 = R^1$ ist und $R^1$ mit Ausnahme von Wasserstoff die in Anspruch 1 angegebene Bedeutung hat und gegebenenfalls zuvor geschützte funktionelle Gruppen gegebenenfalls in die ungeschützten funktionellen Gruppen überführt und im Falle der Herstellung der verschiedenen Salze mit den entsprechenden Säuren umsetzt.

g) $R^1$ und $R^2$ mit Ausnahme von Wasserstoff die in Anspruch 1 angegebene Bedeutung haben, $R^4$ Wasserstoff bedeutet und $R^3$ eine Acetylgruppe oder eine mit einem oder mehreren Halogenatom(en) - untereinander gleich oder verschieden - substituierte Acetylgruppe bedeutet - 1,2,3,4-Tetrahydronaphthaline der allgemeinen Formel XXVII

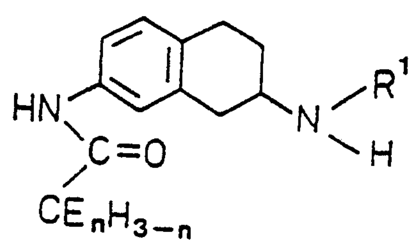

(XXVII)

- in der E für Halogen steht und n eine Zahl 0, 1, 2 oder 3 bedeutet, wobei die Halogene untereinander gleich oder verschieden sein können und $R^1$ mit Ausnahme von Wasserstoff die in Anspruch 1 angegebene Bedeutung hat -

in einem inerten Lösungmittel bzw. Lösungsmittelgemisch gegebenenfalls in Gegenwart einer Base mit Alkylierungsmitteln der allgemeinen Formel XXVIII

G-$R^2$ (XXVIII)

umsetzt, worin G ein Halogen, einen Sulfat-, einen Tosylat-, Methansulfonat- oder einen Halogenmethansulfonatrest oder eine andere durch eine Aminofunktion substituierbare Gruppe bedeutet und $R^2$ mit Ausnahme von Wasserstoff die im Anspruch 1 angegebene Bedeutung hat und gegebenenfalls zuvor geschützte funktionelle Gruppen in andere funktionelle Gruppen überführt und im Falle der Herstellung der verschiedenen Salze mit den entsprechenden Säuren umsetzt.

h) $R^1$, $R^2$ mit Ausnahme von Wasserstoff die im Anspruch 1 angegebene Bedeutung haben und $R^3$ eine gegebenenfalls halogensubstituierte Acetylgruppe und $R^4$ Wasserstoff bedeuten - 1,2,3,4-Tetrahydronaphthalin-Derivate der allgemeinen Formel XXVII unter reduktiven Bedingungen in einem inerten Lösungsmittel gegebenenfalls in Anwesenheit eines Katalysators mit einer Carbonylverbindung der allgemeinen Formel XXIX

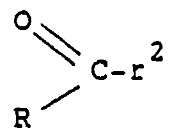

(XXIX)

umsetzt, wobei R Wasserstoff oder eine Alkylgruppe bedeutet und -CHR-$r^2$ = $R^2$ ist und $R^2$ mit Ausnahme von Wasserstoff die in Anspruch 1 angegebene Bedeutung hat und gegebenenfalls zuvor geschützte funktionelle Gruppen überführt und im Falle der Herstellung der verschiedenen Salze mit den entsprechenden Säuren umsetzt.

i) $R^1$, $R^2$ und $R^4$ mit Ausnahme von Wasserstoff die in Anspruch 1 angegebene Bedeutung haben, $R^3$ eine Acetylgruppe oder gegebenenfalls eine mit einem oder mehreren Halogenatom(en) - untereinander gleich oder verschieden - substituierte Acetylgruppe bedeutet - 1,2,3,4-Tetrahydronaphthalin-Derivate der allgemeinen Formel XXX

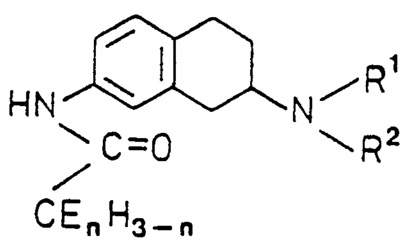

(XXX)

worin E und n die unter g angegebene Bedeutung haben in einem inerten Lösungsmittel bzw. Lösungsmittelgemisch - nach Umsetzung mit einer Base - mit Alkylierungsmitteln der allgemeinen Formel XXXI

G-$R^4$ (XXXI)

umsetzt, worin G ein Halogen einen Sulfat-, einen Tosylat-, einen Methansulfonat- oder einen Halogenmethansulfonatrest oder eine andere durch eine Amidofunktion substituierbare Gruppe bedeutet und $R^4$ mit Ausnahme von Wasserstoff die in Anspruch 1 angegebene Bedeutung hat.

j) $R^1$, $R^2$ und $R^4$ mit Ausnahme von Wasserstoff die in Anspruch 1 angegebene Bedeutung haben und $R^3$ Wasserstoff bedeutet - 1,2,3,4-Tetrahydronaphthalin-Derivate der allgemeinen Formel XXXII

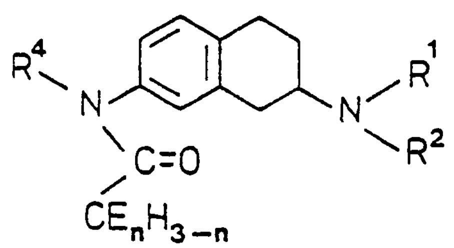

(XXXII)

worin E und n die unter g angegebene Bedeutung haben unter an sich bekannten Reaktionsbedingungen

der Verseifung unterwirft.

Europäisches Patentamt

# EUROPÄISCHER RECHERCHENBERICHT

Nummer der Anmeldung

EP 89 11 7391

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | JOURNAL OF MEDECINAL CHEMISTRY Band 29, Nr. 5, 1986, Seiten 648-654, American Chemical Society; A. A. ASSELIN et al.: "Drug Design via Pharmacophore Identification. Dopaminergic Activity of 3H-Benz(e)indol-8-amines and Their Mode of Interaction with the Dopamine Receptor" * Seiten 650,652-654 * | 1-3,8, 10 | C 07 C 211/60<br>C 07 C 271/30<br>C 07 C 271/24<br>C 07 C 233/34<br>C 07 C 233/43<br>C 07 D 333/20<br>C 07 D 307/52<br>C 07 D 213/36<br>C 07 D 295/16<br>C 07 D 313/20 |
| X | BERICHTE DER DEUTSCHEN CHEMISCHEN GESELLSCHAFT 57. Jahrgang, 1924, Band II, Seiten 1731-1739, "Verlag Chemie", GMBH, Leipzig und Berlin; A. WINDAUS: "Ueber einige Derivate des ac.-Tetrahydro-beta-naphthylamins" * Seiten 1737,1738 * | 1-3,10 | |
| X | BEILSTEINS HANDBUCH DER ORGANISCHEN CHEMIE 4. Ergänzungswerk, 13. Band, 1950-1959, 4. Auflage, Seite 328, Springer-Verlag Berlin, Heidelberg, New York, Tokyo 1985 | 1-3 | **RECHERCHIERTE SACHGEBIETE (Int. Cl.5)** |
| X | EP-A-0 055 043 (AYERST, MCKENNA AND HARRISON INC.) * Seite 28-30; Ansprüche 1,9 * | 1-3 | C 07 C 211/00<br>C 07 C 271/00<br>C 07 C 233/00<br>C 07 D 333/00<br>C 07 D 307/00<br>C 07 D 213/00<br>C 07 D 295/00<br>C 07 C 313/00 |
| Y | | 8-10 | |
| Y | EP-A-0 270 947 (BAYER AG) * Ansprüche 5,6 * -/- | 1-3,8-10 | |

Der vorliegende Recherchenbericht wurde für alle Patentansprüche erstellt

| Recherchenort | Abschlußdatum der Recherche | Prüfer |
|---|---|---|
| BERLIN | 25-10-1989 | RUFET J.M.A. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

Europäisches Patentamt

# EUROPÄISCHER RECHERCHENBERICHT

Nummer der Anmeldung

EP 89 11 7391

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | EP-A-0 041 488 (ASTRA LAEKEMEDEL AKTIEBOLAG)<br>* Zusammenfassung; Ansprüche *<br>--- | 1-3,8-10 | |
| A | EP-A-0 074 903 (SYNTHELABO)<br>* Seiten 17,18; Ansprüche *<br>----- | 1,2,5-10 | |

RECHERCHIERTE SACHGEBIETE (Int. Cl.5)

Der vorliegende Recherchenbericht wurde für alle Patentansprüche erstellt

| Recherchenort | Abschlußdatum der Recherche | Prüfer |
|---|---|---|
| BERLIN | 25-10-1989 | RUFET J.M.A. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)